(19) **Europäisches Patentamt**
**European Patent Office**
**Office européen des brevets**

(11) **EP 3 067 029 B1**

(12) **EUROPEAN PATENT SPECIFICATION**

(45) Date of publication and mention
of the grant of the patent:
**01.11.2017 Bulletin 2017/44**

(51) Int Cl.:
*A61F 13/495* *(2006.01)*    *A61F 13/472* *(2006.01)*
*A61F 13/49* *(2006.01)*    *A61F 13/537* *(2006.01)*

(21) Application number: **15158496.8**

(22) Date of filing: **10.03.2015**

(54) **Absorbent articles with structures able to lift upwards towards the wearer**

Am Körper eines Trägers festziehbare saugfähige Artikel

Articles absorbants avec des structures capable de se soulever vers le haut en direction de l'utilisateur

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**14.09.2016 Bulletin 2016/37**

(73) Proprietor: **The Procter & Gamble Company
Cincinnati, OH 45202 (US)**

(72) Inventor: **Liebe, Tina
65824 Schwalbach am Taunus (DE)**

(74) Representative: **Heide, Ute
Procter & Gamble Service GmbH
IP Department
Frankfurter Strasse 145
61476 Kronberg im Taunus (DE)**

(56) References cited:
**EP-A1- 1 132 066         EP-A2- 0 985 395
WO-A2-95/17150         US-A1- 2005 074 584
US-A1- 2005 267 434     US-A1- 2006 142 723**

**Description**

FIELD OF THE INVENTION

The invention relates to personal hygiene absorbent articles for wearing in the lower torso, such as but not limited to diapers and training pants for babies, toddlers and adults, or sanitary napkins. More particularly, the invention relates to absorbent articles wherein the topsheet is able to lift away from the absorbent core in use due to a structure below the topsheet which can increase in caliper. Alternatively, the structure may be positioned on the wearer-facing surface of the topsheet such that when the structure increases in caliper, it provides improved contact with the skin of the wearer.

BACKGROUND OF THE INVENTION

Over the recent decades, the number of people who are overweight has considerably increased. For example, in the U.S. alone, more than 1/3 of adult females are now considered obese (BMI > 30). The body mass index (BMI) is a classification system for body shapes based upon height and mass. BMI can be calculated as follows:

$$BMI = \frac{weight(kg)}{height(m)^2}$$

The index comprises different classes of body mass, including: underweight (BMI < 20), normal weight (BMI 20-25), overweight (BMI 25-30), obese (BMI 30-40), and morbidly obese (BMI > 40).

As BMI increases and body shape changes, it was discovered that the shape/morphology of the intimate region through the crotch also changes. Based on this insight, there are several mechanistic explanations as to why performance of absorbent articles often degrades as BMI increases. For instance, there exists a higher probability of fat folds / skin creases for overweight and especially obese wearer's which can attract the fluid (capillarity) and trap it once there. Additionally, there is a higher probability of reduced space between the tights of such wearers, specifically in the crotch region.

As a consequence, today's absorbent articles often struggle to deliver superior protection for obese people. Often, due to the body shape of obese people, absorbent articles fail to provide close contact with the skin of the wearer. Hence, spreading of body fluids is more likely to happen. Spreading occurs when fluid moves along the body or when fluid is trapped in the interface between the wearer's body and the absorbent article. This can be uncomfortable for the wearer, leading to a feeling of uncleanliness and insecurity, and increases the risk of leakage of body fluids. There is hence a desire to minimize spreading of urine or menstrual fluid.

Consequently, there is a need for absorbent articles, which provide improved close contact with the skin of the wearer to reduce the occurrence of spreading of urine and feces.

US 2005/267434 relates to a sanitary napkin including a napkin body containing a liquid absorbent layer and a three-dimensional structure is disposed on the skin-side surface of the napkin body. The three-dimensional structure is formed of a reinforcing member and a liquid-permeable sheet, and an elastic member is disposed along an apex to extend in a longitudinal direction. The reinforcing member has high-density portions and low-density portions alternating with each other in the longitudinal direction so that the apex can be shortened in the longitudinal direction by an elastic contractive force of the elastic member. Therefore, the napkin body can be easily kept curved.

WO 95/17150 A2 discloses to a sanitary napkin having a lifting member with a plurality of pleats. The pleats can be formed by differentially elongating elastic elements and joining the elastic elements at spaced apart locations along their lengths. The pleats have a Z-direction height for providing Z-direction displacement of a portion of the topsheet relative to the backsheet.

US 2005/074584 is concerned with liner composites suitable for incorporation into disposable absorbent articles. The liner composites include an extensible liner material and a non-tensioned elastic. The liner composites are adapted to attain three-dimensional configurations upon activation.

EP 0 985 395 A1 discloses a sanitary napkin including therein an elastic member longitudinally extending under tension along a center line dividing a width of the napkin. An upper portion of the napkin protrudes upward as the elastic member contracts.

US 2006/142723 relates to a sanitary napkin including: a napkin body having first and second projections each exerting an elastic contractive force between longitudinally opposing front and rear ends to concavely curve the body surface of the napkin body and raise itself from the body surface of the napkin body.

EP 1 132 066 discloses an absorbent article including a support body able to bent in the longitudinal direction by the tension of an elastic member, and an liquid absorbing member deforming to bulge at its widthwise central portion away from the support body.

SUMMARY OF THE INVENTION

An absorbent article is provided, having a longitudinal dimension and a transverse dimension and comprising a liquid pervious topshcct, a liquid impervious backsheet; and an absorbent core disposed between the topsheet and the backsheet. The absorbent article further comprises a structure which:

may be positioned above the absorbent core and underneath the topsheet wherein the structure is attached to the component of the absorbent article underneath the structure; or

may be positioned above the topsheet and be attached to the wearer-facing surface of the topsheet.

The structure has a longitudinal dimension, a lateral dimension and a caliper. One or more elastic elements are either comprised by the structure, are associated with the structure, or both.

The structure is in a flat configuration when the absorbent article is flattened out, wherein the one or more elastic elements are stretched along the longitudinal dimension of the structure. Contraction of the one or more elastic elements applies a force along the longitudinal dimension of the structure, thus converting the structure into an erected, three-dimensional configuration. The structure has a higher caliper in its erected configuration compared to the caliper of the structure in its flat configuration. If the structure is positioned below the topsheet, the erected structure spaces the topsheet away from the absorbent core. If the structure is positioned above the topsheet, the distal end of the structure is spaced away from the topsheet when the structure is in its erected configuration.

The structure may be comprised by the crotch region of the absorbent article and may or may not extend into the first and/or second waist regions.

The absorbent article may further comprise an acquisition system, the acquisition system being positioned underneath the structure, and underneath the optional secondary topsheet, and above the absorbent core. Alternatively, the absorbent article may further comprise an acquisition system, wherein the acquisition system is positioned above the structure and below the topsheet. Still further alternatively, the absorbent article may further comprise an acquisition system, wherein the acquisition system has two or more layers, wherein one or more layer are positioned above the structure and underneath the topsheet, and one or more further layers of the acquisition system may be positioned underneath the structure and above the absorbent core. In a still further alternative, the absorbent article does not have an acquisition system.

The topsheet may made of extensible material, or highly extensible material, in order not to hinder erection of the structure. The topsheet may be be made of non-elastic material.

The lateral dimension of the structure may be parallel to the longitudinal dimension of the absorbent article. Alternatively, the longitudinal dimension of the structure may be parallel to the longitudinal dimension of the absorbent article.

The non-clastic materials comprised by the structure may have a bending stiffness of less than 500 mNm, preferably less than 400 mNm, more preferably less than 200 mNm. The non-clastic materials comprised by the structure may have a tensile strength of less than 80 N/cm, preferably less than 50 N/cm, more preferably less than 40 N/cm.

The structure comprises a first and a second layer, each layer having an inner and an outer surface, a longitudinal dimension parallel to the longitudinal dimension of the structure confined by first and second spaced apart lateral edges, and a lateral dimension parallel to the lateral dimension of the structure confined by two spaced apart longitudinal edges. The first and second layer at least partly overlap each other, wherein the second layer is able to shift relative to the first layer along the longitudinal structure dimension upon release of a contractive force, which has provided by the stretched one or more elastic elements along the longitudinal dimension. The structure further comprises ligaments provided between the first and second layer in at least a part of the region where the first and second layer overlap each other. Each ligament has a longitudinal dimension confined by two spaced apart lateral ligament edges, and a lateral dimension confined by two spaced apart longitudinal ligament edges. Each ligament is attached to the inner surface of the first layer with a portion at or adjacent to one of the ligament's lateral edges in a first ligament attachment region and is attached to the inner surface of the second layer with a portion at or adjacent to the ligament's other lateral edge in a second ligament attachment region, the region of each ligament between the first and second ligament attachment region forming a free intermediate portion. The ligaments are spaced apart from one another along the longitudinal dimension of the structure, and the attachment of all ligaments to the first and second layer in the first and second ligament attachment regions is such that the free intermediate portions of the ligaments are able to convert from an initial flat configuration to an erected configuration upon release of a contractive force, which has provided by the stretched one or more elastic elements along the longitudinal dimension of the structure, thus converting the structure as a whole from an initial flat configuration into an erected configuration, wherein the erection is in the direction perpendicular to the longitudinal and the lateral dimension of the structure, thus increasing in caliper.

The longitudinal dimension of the ligaments in the initial flat configuration of structure may be substantially parallel with the longitudinal dimension of the first and second layer.

One or more ligaments provided towards the longitudinal center of the structure may have a higher tensile strength and/or a higher bending stiffness than the ligaments provided towards the lateral edges of the structure.

In one or more ligaments, those portions of the free intermediate portion which are directly adjacent to the first and/or second ligament attachment region may have a different bending stiffness, different tensile strength, or different bending stiffness and tensile strength than the remaining free intermediate portion of said ligament.

The first layer and the second layer may be made of a single continuous material which is folded over at one of the lateral edges of the structure, such that one of the lateral edges of the first layer is coincident with one of the lateral edges of the second layer, with these lateral edges being located at the interface of the first and second layer.

For all ligaments in the structure, a first surface of the free intermediate portion may face towards the inner surface of the first layer and a second surface of the free intermediate portion may face towards the inner surface second layer in the structure's flat configuration, and the first surface of the free intermediate portion may face towards the second surface of the neighboring ligament when the structure is in the erected configuration.

For all ligaments in the structure, the ligament's first surface may not be facing towards the inner surface of the second layer when the structure is in its initial flat configuration, and the ligament's second surface may not be facing towards the inner surface of the first layer when the structure is in its initial flat configuration.

The ligaments of the structure may have a tensile strength of from 1N/cm to 100N/cm and/or may have a bending stiffness of from 0.01mNm to 1Nm.

BRIEF DESCRIPTION OF THE DRAWINGS

These and other features, aspects and advantages of the present invention will become better understood with regard to the following description, appended claims, and accompanying drawing where:

Fig. 1A is a side view (parallel to the lateral dimension) of of a cell-forming structure - with ligaments having Z-like shape, wherein the structure is in its initial flat configuration and wherein the free intermediate portion of all ligaments has the same length.

Fig. 1B is a side view of the embodiment of Fig. 1A, wherein the structure is now in its erected configuration

Fig. 2 is a side view (parallel to the lateral dimension) of a cell-forming structure - with ligaments having Z-like shape -, wherein the structure is in its erected configuration and wherein the free intermediate portion of the ligaments varies with the central ligament having the biggest length

Fig. 3 is a side view (parallel to the lateral dimension) of a cell-forming structure - with ligaments having Z-like shape -, wherein the structure is in its erected configuration and wherein the free intermediate portion of the ligaments varies with the ligament towards one lateral edge of the structure having the biggest length

Fig. 4A is a side view (parallel to the lateral dimension) of a cell-forming structure - with ligaments having Z-like shape, wherein the structure is in its initial flat configuration and wherein the structure comprises a layer-to-layer stop aid

Fig. 4B is a side view (parallel to the lateral dimension) of the embodiment of Fig 4A, now in its erected configuration.

Fig. 5A is a side view (parallel to the lateral dimension) of a cell-forming structure - with ligaments having Z-like shape, wherein the structure is in its initial flat configuration and wherein the structure comprises a layer-to-ligament stop aid

Fig. 5B is a side view (parallel to the lateral dimension) of the embodiment of Fig 5A, now in its erected configuration

Fig. 6 is a side view of the structure shown in Fig. 1B which comprises a ligament-to-ligament material between neighboring ligaments

Fig. 7 is a side view of a cell-forming structure - with ligaments having Z-like shape

Fig. 8A is a side view of a cell-forming structure -with ligaments having C-like shape, wherein the structure is in its initial flat configuration

Fig. 8B is a side view of the embodiment of Fig. 8A, wherein the structure is in its partly erected configuration

Fig. 8C is a side view of the embodiment of Fig. 8A, wherein the structure is in its maximum erected configuration

Fig. 9A is a side view of a cell-forming structure -with ligaments having Double-T-like shape-, wherein the structure is in its initial flat configuration

Fig. 9B is a side view of the embodiment of Fig. 9A, wherein the structure is in its partly erected configuration

Fig. 9C is a side view of the embodiment of Fig. 9A, wherein the structure is in its maximum erected configuration

Fig. 10A is a side view of a cell-forming structure -with ligaments having T-like shape-, wherein the structure is in its initial flat configuration

Fig. 10B is a side view of the embodiment of Fig. 10A, wherein the structure is in its partly erected configuration

Fig. 10C is a side view of the embodiment of Fig. 10A, wherein the structure is in its maximum erected configuration

Fig. 11A is a side view of a cell-forming structure - with ligaments of Z-like shape having inverse configuration, wherein the structure is in its initial flat configuration

Fig. 11B is a side view of the embodiment of Fig. 11A, wherein the structure is in its erected configuration

Fig. 12A is a side view of a cell-forming structure - with ligaments of C-like shape having inverse configuration, wherein the structure is in its initial flat configuration

Fig. 12B is a side view of the embodiment of Fig. 12A, wherein the structure is in its erected configuration

Fig. 13 is a side view of a cell-forming structure not comprised by the present invention, wherein the structure cannot be converted into an erected configuration

Fig. 14A is a side view of a cell-forming structure -with ligaments made of two-layered laminates and having Double-T-like shape-, wherein the structure is in its initial flat configuration

Fig. 14B is a side view of the embodiment of Fig. 14A, wherein the structure is in its partly erected configuration

Fig. 14C is a side view of the embodiment of Fig. 14A, wherein the structure is in its maximum erected configuration

Fig. 15A is a side view of a cell-forming structure -with ligaments compiled of separate pieces of material and having Double-T-like shape-, wherein the structure is in its initial flat configuration

Fig. 15B is a side view of the embodiment of Fig. 15A, wherein the structure is in its partly erected configuration

Fig. 15C is a side view of the embodiment of Fig. 15A, wherein the structure is in its maximum erected configuration

Fig. 16A is a side view of a cell-forming structure -with ligaments compiled of separate pieces of material and having Z-like shape-, wherein the structure is in its initial flat configuration

Fig. 16B is a side view of the embodiment of Fig. 16A, wherein the structure is in its partly erected configuration

Fig. 16C is a side view of the embodiment of Fig. 16A, wherein the structure is in its maximum erected configuration

Fig. 17A is a side view of a cell-forming structure -with ligaments having cut out areas and having Z-like shape-, wherein the structure is substantially in its initial flat configuration

Fig. 17B is a side view of the embodiment of Fig. 17A, wherein the structure is in its erected configuration

Fig. 18 is a schematic drawing of parts of the equipment used for the modulus test method

Fig. 19 is a schematic drawing of a structure comprising an elastic member, wherein the structure is in its flat configuration

Fig. 20 is a schematic drawing of the embodiment of Fig. 19, wherein the structure is in its erected configuration.

Fig. 21 is a spatial representation of the structure of Fig. 20

Fig. 22 is a schematic drawing of an alternative structure to the structure shown in Fig. 19 and comprising an elastic member, wherein the structure is in its flat configuration

Fig. 23 is a schematic drawing of the embodiment of Fig. 22, wherein the structure is in its erected configuration.

Fig. 24 is a spatial representation of the structure of Fig. 23

Fig. 25 is a schematic drawing of an alternative structure to the structure shown in Fig. 22 and comprising an elastic member, wherein the structure is in its flat configuration. Compared to the structure of Fig. 22, the ligaments are spaced apart from each other at a wider distance along the longitudinal dimension of the structure

Fig. 26 is a schematic drawing of a structure placed below the topsheet and above the acquisition system of an absorbent article.

Fig. 27 is a top view of an exemplary absorbent article in the form of a diaper.

DETAILED DESCRIPTION OF THE INVENTION

**Definitions**

"Absorbent article" refers to devices that absorb and contain body exudates, and, more specifically, refers to devices that are placed against or in proximity to the body of the wearer to absorb and contain the various exudates discharged from the body. Absorbent articles may include diapers (baby diapers and diapers for adult incontinence), pants, inserts, feminine care absorbent articles such as sanitary napkins or pantiliners, and the like. As used herein, the term "exudates" includes, but is not limited to, urine, blood, vaginal discharges, sweat and fecal matter. Preferred absorbent articles of the present invention are disposable absorbent articles, more preferably disposable diapers and disposable pants.
"Disposable" is used in its ordinary sense to mean an article that is disposed or discarded after a limited number of usage over varying lengths of time, for example, less than 20 usages, less than 10 usages, less than 5 usages, or less than 2 usages. If the disposable absorbent article is a diaper, a pant, sanitary napkin, sanitary pad or wet wipe for personal hygiene use, the disposable absorbent article is most often intended to be disposed after single use.
"Diaper" and "pant" refers to an absorbent article generally worn by babies, infants and incontinent persons about the lower torso so as to encircle the waist and legs of the wearer and that is specifically adapted to receive and contain urinary and fecal waste. In a pant, as used herein, the longitudinal edges of the first and second waist region are attached to each other to a pre-form waist opening and leg openings. A pant is placed in position on the wearer by inserting the wearer's legs into the leg openings and sliding the pant absorbent article into position about the wearer's lower torso. A pant may be pre-formed by any suitable technique including, but not limited to, joining together portions of the absorbent article using refastenable and/or non-refastenable bonds (e.g., seam, weld, adhesive, cohesive bond, fastener, etc.). A pant may be preformed anywhere along the circumference of the article (e.g., side fastened, front waist fastened). In a diaper, the waist opening and leg openings are only formed when the diaper is applied onto a wearer by (releasable) attaching the longitudinal edges of the first and second waist region to each other on both sides by a suitable fastening system.
The term "film" as used herein refers to a substantially non-fibrous sheet-like material wherein the length and width of the material far exceed the thickness of the material. Typically, films have a thickness of about 0.5 mm or less. Films may be configured to be liquid impermeable and/or vapor permeable (i.e., breathable). Films may be made of polymeric, thermoplastic material, such as polyethylene, polypropylene or the like.
"Garment-facing surface" means the outermost portion of an element of a wearable absorbent article when the absorbent article is worn as intended. The opposing surface, or innermost portion, of the same element is referred to as the "wearer-facing surface." It is to be understood that the garment-facing surface and the wearer-facing surface of an element are relative to the wearer of the article with the garment-facing surface being furthest from the wearer and the wearer-facing surface being closest to the wearer. In the example of a typical disposable diaper, the portion of the outer cover that faces away from the wearer is the garment-facing surface while the opposing surface of the outer cover is the wearer-facing surface.

"Longitudinally inboard" and "longitudinally outboard" as used herein in relation to the absorbent article describes the position of a component or material relative to another component or material with respect to the longitudinal axis of the absorbent article. Longitudinally inboard means closer to the longitudinal axis whereas longitudinally outboard means further away from the longitudinal axis.

"Non-extensible" as used herein refers to a material which, upon application of a force, elongates beyond its original length by less than 20 % if subjected to the following test: A rectangular piece of the material having a width of 2.54 cm and a length of 25.4 cm is maintained in a vertical position by holding the piece along its upper 2.54 cm wide edge along its complete width. A force of 10 N is applied onto the opposite lower edge along the complete width of the material for 1 minute (at 25°C and 50% rel. humidity; samples should be preconditioned at these temperature and humidity conditions for 2 hours prior to testing). Immediately after one minute, the length of the piece is measured while the force is still applied and the degree of elongation is calculated by subtracting the initial length (25.4 cm) from the length measured after one minute.

If a material elongates beyond its original length by more than 20 % if subjected to the above described test, it is "extensible" as used herein.

"Highly non-extensible" as used herein refers to a material, which, upon application of a force, elongates beyond its original length by less than 10% if subjected to the test described above for "non-extensible" material.

"Non-elastic" as used herein refers to a material which does not recover by more than 20% if subjected to the following test, which is to be carried out immediately subsequent to the test on "non-extensibility" set out above.

Immediately after the length of the rectangular piece of material has been measured while the 10N force is still applied, the force is removed and the piece is laid down flat on a table for 5 minutes (at 25°C and 50% rel. humidity) to be able to recover. Immediately after 5 minutes, the length of the piece is measured again and the degree of elongation is calculated by subtracting the initial length (25.4 cm) from the length after 5 minutes.

The elongation after one minute while the force has been applied (as measured with respect to "non-extensibility") is compared to the elongation after the piece has been laid down flat on a table for 5 minutes: If the elongation does not recover by more than 20%, the material is considered to be "non-elastic".

If a material recovers by more than 20%, the material is considered "elastic" as used herein.

"Highly non-elastic" as used herein refers to a material, which is either "non-extensible" or which does not recover by more than 10% if subjected to the test set out above for "non-elastic".

For use in the cell forming structures of the present invention, extensible, non-extensible, highly non-extensible, elastic, non-elastic and highly non-elastic relate to the dimension of the material, which, once the material has been incorporated into the structure, is parallel to the longitudinal dimension of the structure. Hence, the sample length of 25.4 cm for carrying out the tests described above corresponds to the longitudinal dimension of the cell forming structure once the material has been incorporated into the structure.

A "nonwoven web" is a manufactured web of directionally or randomly oriented fibers, consolidated and bonded together. The term does not include fabrics which are woven, knitted, or stitch-bonded with yarns or filaments. The fibers may be of natural or man-made origin and may be staple or continuous filaments or be formed in situ. Commercially available fibers have diameters ranging from less than about 0.001 mm to more than about 0.2 mm and they come in several different forms: short fibers (known as staple, or chopped), continuous single fibers (filaments or monofilaments), untwisted bundles of continuous filaments (tow), and twisted bundles of continuous filaments (yarn). Nonwoven fabrics can be formed by many processes such as meltblowing, spunbonding, solvent spinning, electrospinning, and carding. Nonwoven webs may be bonded by heat and/or pressure or may be adhesively bonded. Bonding may be limited to certain areas of the nonwoven web (point bonding, pattern bonding). Nonwoven webs may also be hydro-entangled or needle-punched. The basis weight of nonwoven fabrics is usually expressed in grams per square meter ($g/m^2$).

A "pantiliner" and a "sanitary napkin" generally have two end regions and a middle region (i.e. a crotch region). The pantiliner and the sanitary napkin has a body-facing surface and a garment facing surface. The size and shape of the absorbent structure positioned between the topsheet and the backsheet can be altered to meet absorbent capacity requirements, and to provide comfort to the wearer. The garment facing surface of the pantiliner and of the sanitary napkin can have thereon pressure sensitive adhesive for affixing to a wearer's undergarments. Typically, such adhesive is covered with a release strip which is removed before affixing to the undergarment. Pantiliners can also be provided with lateral extensions known commonly in the art as "flaps" or "wings" intended to extend over and cover the panty elastics in the crotch region of the user's undergarment. However, wings are normally not used with pantiliners but are more often used in sanitary napkins.

A "paper" refers to a wet-formed fibrous structure comprising cellulose fibers.

"Releasably attached", as used herein, refers to a temporary attachment. Temporary attachment can be achieved, e.g. by using an adhesive which allows for release of the attachment by use of moderate force, such as can be applied by a person without application of undue forces and leading to rupturing or substantial damaging (affecting proper and intended use) of the absorbent article. Releasably attachment can also be achieved by using an adhesive which provides strong attachment initially when the adhesive is applied, e.g. during manufacturing of the absorbent article, and wherein the adhesive forces decrease over time, e.g. by evaporation of certain solvents comprised by the adhesive during application of the adhesive or by degradation of certain ingredients comprised by the adhesive. Such adhesives may become brittle over time. It should be noted that though such releasable attachments may, under certain conditions, already lead to detachment prior to a consumer using the absorbent article (e.g. by removing it from a package prior to use), the releasable attachment will nevertheless be useful in the present invention, as long as the releasable attachment provides for intial attachment, e.g. during manufacture and packaging of the absorbent article. Unless specifically mentioned herein that an attachment/bond is meant to be releasable, all attachments/bonds described herein are permanent, i.e. they do not detach and open under conditions to which an absorbent article is normally subjected during its lifetime.

"Sheet-like foam", as used herein is a solid sheet that is formed by trapping pockets of gas. The solid foam may be closed-cell foam or open-cell foam. In closed-cell foam, the gas forms discrete pockets, each completely surrounded by the solid material. In open-cell foam, the gas pockets connect with each other. "Sheet-like" means that the length and width of the material far exceed the thickness of the material

"Transversally inboard" and "transversally outboard" as used herein in relation to the absorbent article describes the position of a component or material relative to another component or material with respect to the transversal axis of the absorbent article. Transversally inboard means closer to the transversal axis whereas transversally outboard means further away from the transversal axis.

The terms "upper", "above", "on top of", as used herein in respect of the absorbent article relates to a position on a wearer-facing surface. Likewise, the terms "lower", "below", "beneath", "underneath" and "under" as used herein in respect of the absorbent article relates to a position on a garment-facing surface.

### Disposable absorbent articles

The structures of the present invention can find a wide variety of applications in absorbent articles, such as disposable absorbent articles.

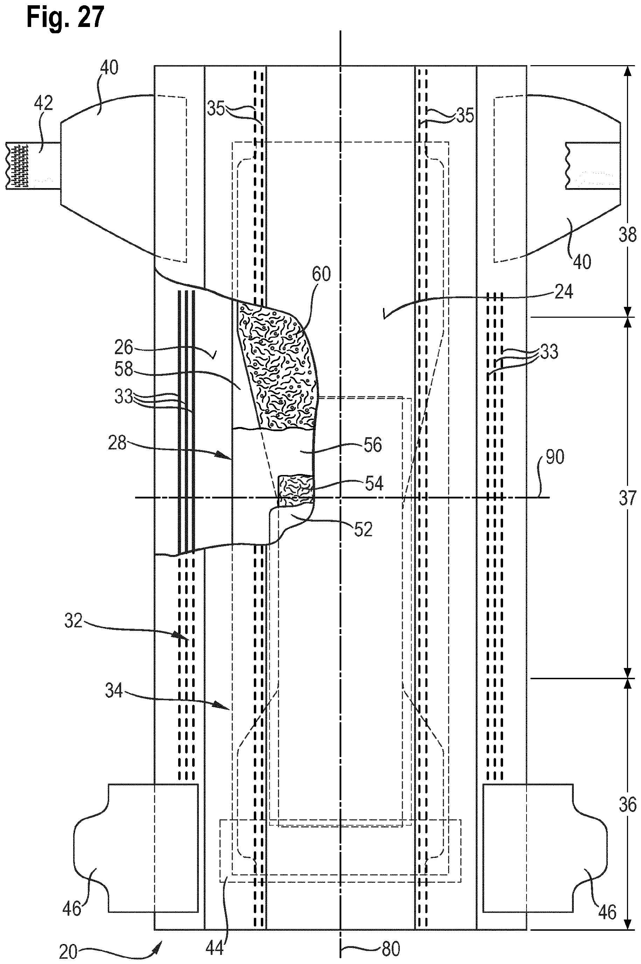

A typical absorbent article is represented in Fig. 27 in the form of a diaper 20. However, the structure may be applied in other disposable absorbent articles, such as pants, pantiliners and sanitary napkins in the same manner and configuration as described herein below for a diaper.

In more details, Figure 27 is a plan view of an exemplary diaper 20, in a flat-out state, with portions of the diaper being cut-away to more clearly show the construction of the diaper 20. As said, this diaper 20 is shown for illustration purpose only as the structure of the present invention may be comprised in a wide variety of diapers or other absorbent articles.

As shown in Figure 27, the absorbent article, here a diaper, can comprise a liquid pervious topsheet 24, a liquid impervious backsheet 26, an absorbent core 28 which is preferably positioned between at least a portion of the topsheet 24 and the backsheet 26. The absorbent core 28 can absorb and contain liquid received by the absorbent article and may comprise absorbent materials 60, such as superabsorbent polymers and/or cellulose fibers, as well as other absorbent and non-absorbent materials commonly used in absorbent articles (e.g. thermoplastic adhesives immobilizing the superabsorbent polymer particles). The diaper 20 may also include optionally an acquisition system with an upper 52 and lower 54 acquisition layer.

The absorbent article may comprise a fastening system, such as an adhesive fastening system or a hook and loop fastening member, which can comprise tape tabs 42, such as adhesive tape tabs or tape tabs comprising hook elements, cooperating with a landing zone 44 (e.g. a nonwoven web providing loops in a hook and loop fastening system).

The diaper 20 as shown in Figure 27 can be notionally divided in a first waist region 36, a second waist region 38 opposed to the first waist region 36 and a crotch region 37 located between the first waist region 36 and the second waist region 38. The longitudinal centerline 80 is the imaginary line separating the diaper along its length in two equal halves. The transversal centerline 90 is the imagery line perpendicular to the longitudinal line 80 in the plane of the flattened out diaper and going through the middle of the length of the diaper. The periphery of the diaper 20 is defined by the outer edges of the diaper 20. The longitudinal edges of the diaper may run generally parallel to the longitudinal centerline 80 of the diaper 20 and the end edges run between the longitudinal edges generally parallel to the transversal centerline 90 of the diaper 20. The crotch region and the first and second waist region each constitute 1/3 of the absorbent article along the longitudinal centerline.

Further, the diaper may comprise other elements, such as a back waist feature, which may be non-elastic or elastic, and a front waist feature, which may be non-elastic or elastic, a lotion applied onto the wearer-facing surface of the topsheet, back ears 40, and/or front ears 46. The front and/or back ears 40, 46 may be separate components attached to the diaper or may instead be continuous with portions of the topsheet and/or backsheet-and/or even portions of the absorbent core - such that these portions form all or a part of the front and/or back ears 40, 46. Also combinations of the aforementioned are possible, such that the front and/or back ears 40, 46 are formed by portions of the topsheet and/or backsheet while additional materials are attached to form the overall front and/or back ears 40, 46. The front and/or back ears may be elastic or non-elastic.

The topsheet 24, the backsheet 26, and the absorbent core 28 may be assembled in a variety of well known configurations, in particular by gluing or heat embossing. Exemplary diaper configurations are described generally in US3,860,003; US5,221,274; US5,554,145; US5,569,234; US5,580,411; and US6,004,306.

As the structure has a relatively low caliper when in its initial flat configuration, it does not significantly contribute to the volume and bulk of the absorbent article prior to use. Hence, the structures do not add significantly to the overall packaging and storage volume of the absorbent articles.

The absorbent article may also comprise barrier leg cuffs 34 disposed adjacent to each of the longitudinal edges of the absorbent article and extending substantially parallel to the longitudinal dimension of the article. Each barrier leg cuff 34 is typically delimited by a free standing distal edge and is attached to the rest of the article at a so-called proximal edge. An elastic element within the barrier leg cuffs can advantageously be present for spacing the distal edge away from the body-facing surface of the article so that the barrier leg cuffs can stand up when worn by a user. The elastic element can be for example one two or more strands of elastic material 35 attached in a fold towards the distal edge and formed by a nonwoven barrier material. The barrier leg cuffs 34 can provide improved containment of liquids and other body exudates approximately at the junction of the torso and legs of the wearer.

The absorbent article may further comprise a gasketing cuff 32 along each of the longitudinal edges of the article. The gasketing cuff 32 is typically positioned longitudinally outboard of the barrier leg cuff. The gasketing cuff may comprise one or more elastic elements, such as elastic strands. Due to the one or more elastic elements 33, the gasketing cuff is made elastically contractible. The gasketing cuff may further comprise portions of the backsheet, of the topsheet and/or of a nonwoven gasketing material. The nonwoven gasketing material may be the same or similar to the nonwoven barrier material. The nonwoven gasketing material may be coextensive and continuous with the nonwoven barrier material. The one or more elastic elements may be comprised inbetween the portions of the backsheet, of the topsheet and/or of a nonwoven gasketing material.

### Structure below topsheet able to increase in caliper

The absorbent article further comprises a structure, which may be positioned above the absorbent core and underneath the topsheet. The structure is attached to the component of the absorbent article, which is underneath the structure. The structure may comprise one or more elastic elements (195, 196), alternatively or in addition, the structure may be associated with one or more elastic elements (not shown).

The structure has a longitudinal dimension, a lateral dimension and a caliper, wherein the caliper is perpendicular to the longitudinal and lateral dimension. The structure is in a flat configuration when the absorbent article is flattened out (e.g. by lying it flat on a table) and when the one or more elastic elements are stretched along the longitudinal dimension of the structure. In the flat configuration, the caliper of the structure is typically relatively thin, such that the caliper of the flat structure may not contribute considerably to the over all caliper of the absorbent article.

Contraction of the one or more elastic elements applies a force along the longitudinal dimension of the structure, which leads to conversion of the structure from its flat configuration into an erected, three-dimensional configuration. The caliper of the structure increases and thereby spaces the topsheet upwards towards the skin of the wearer in use. The topsheet is hence spaced from the components underneath the structure, e.g. from the absorbent core.

To enable unobstructed conversion of the structure from its flat configuration into its erected configuration, the topsheet may be able to elongate in the longitudinal and/or transverse dimension of the absorbent article, i.e. the topsheet may be made of extensible material. A suitable topsheet may comprise slits, wherein the slits may be distributed over the complete surface of the topsheet or may be provided at least in the area where the structure is positioned. Upon erection of the structure, the slits in the topsheet are able to open up and form apertures, and the topsheet thus elongates in its longitudinal and/or lateral dimension. Alternatively, the topsheet may be formed with folds at least in the area where the structure is positioned. Upon erection of the structure, the folds in the topsheet unfold, and the topsheet elongates in its longitudinal and/or lateral dimension. In still another alternative, the topsheet may be made of extensible material, such as extensible nonwoven. The material may also be rendered extensible, i.e. by incrementally stretching the material (so-called "ring-rolling"). The topsheet may or may not be attached to the structure.

However, it is less desirable that the topsheet is made of elastic material. While such material can undergoe elastic elongation while it is stretched due to erection of the structure, the topsheet may, due to its tendency to retract, exert pressure onto the structure below the topsheet, which may lead to a decrease in caliper of the structure.

Below the structure a secondary topsheet may be placed. Also, the absorbent article may further comprise an acquisition system, the acquisition system being positioned underneath the structure (and underneath an optional secondary topsheet) and above the absorbent core.

Alternatively, the acquisition system or one or more upper layers of the acquisition system may be positioned above the structure and below the topsheet. In such absorbent articles, the acquisition system or those layers of the acquisition system positioned above the structure may also need to enable unobstructed conversion of the structure from its flat configuration into its erected configuration. The acquisition system or the one or more layers of the acquisition system lying above the structure may thus be able to elongate in the longitudinal and/or transverse dimension of the absorbent article. A suitable acquisition material may comprise slits, at least in the area where the structure is positioned. Upon erection of the structure, the slits in the acquisition material are able to open up and form apertures, and the acquisition material thus elongates in its longitudinal and/or lateral dimension. Alternatively, the acquisition material may be formed with folds at least in the area where the structure is positioned. Upon erection of the structure, the folds in the acquisition material unfold, and the acquisition material elongates in its longitudinal and/or lateral dimension. In still another alternative, the acquisition material may be made of extensible material, such as extensible nonwoven. However, it is less desirable that the acquisition material is made of elastic material for the same reasons as given above for the topsheet. Still further alternatively, the acquisition system or those layers of the acquisition system lying above the structure may be lifted upwardly when the structure is converted into its erected configuration. In such instances, the acquisition system or the layers of the acquisition system lying above the structure may not be attached to the topsheet and/or the components beneath the structure, but may, however, be attached to the distal end of the structure (such as to the second layer of a cell-forming structure as described above). In such cases, the acquisition system may have the same longitudinal and lateral dimension as the structure or may only be slightly larger than the structure.

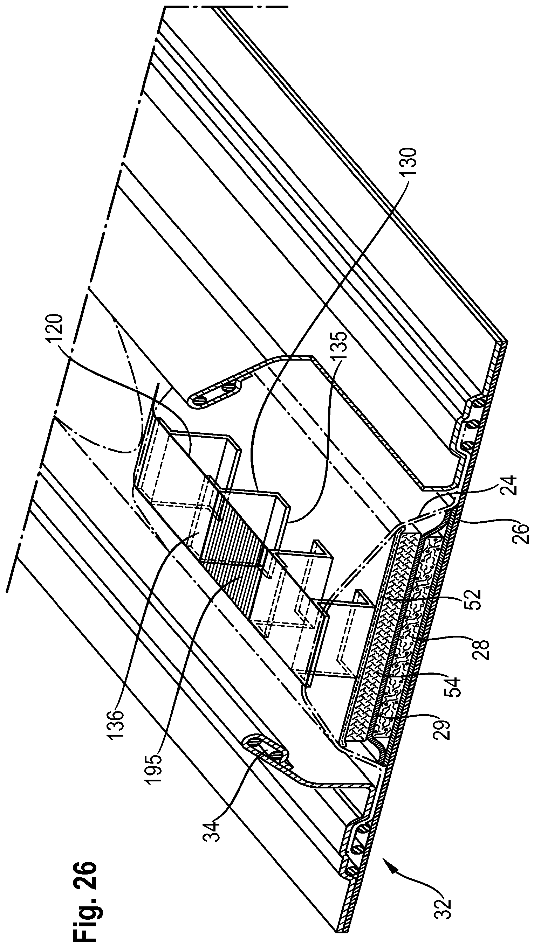

Fig. 26 shows a part of an absorbent article (a diaper), where the structure has been positioned below the topsheet 24 and above the acquisition system 52, 54. In the illustrated example, the structure is a so-called cell-forming structure (described in detail below) and the first layer of this structure (forming the proximal end of the structure) is formed by a portion of the acquisition system's upper layer 52. An elastic element 195 is comprised by the structure's distal end, specifically; an elastic element 195 is comprised by the second layer 120 of the cell forming structure in the region between two neighboring ligaments 130. The structure in Fig. 26 is placed below the topsheet such that the longitudinal dimension of the structure is parallel with the longitudinal axis of the absorbent article. In fact, the structure extends along the longitudinal axis of the absorbent article.

**Structure able to increase in caliper and placed above the topsheet**

Alternatively to placing the structure below the topsheet, the structure may also be placed above the topsheet and attached to the wearer-facing surface of the topsheet. The structure may comprise one or more elastic elements (195, 196), alternatively or in addition, the structure may be associated with one or more elastic elements (not shown).

The structure has a longitudinal dimension, a lateral dimension and a caliper, wherein the caliper is perpendicular to the longitudinal and lateral dimension. The structure is in a flat configuration when the absorbent article is flattened out (e.g. by lying it flat on a table) and when the one or more elastic elements are stretched along the longitudinal dimension of the structure. In the flat configuration, the caliper of the structure is typically relatively thin, such that the caliper of the flat structure may not contribute considerably to the over all caliper of the absorbent article.

Contraction of the one or more elastic elements applies a force along the longitudinal dimension of the structure, which leads to conversion of the structure from its flat configuration into an erected, three-dimensional configuration. The caliper of the structure increases and thereby the distal end of the structure is lifted towards the skin of the wearer.

If the structure is placed above the topsheet, the structure may be, at least partly, enwrapped by a wrap-sheet, such as a nonwoven or a film. The wrap-sheet may enwrap the structure along the longitudinal or along the lateral dimension of the structure. The wrap-sheet may have any shape and may for example be rectangular, rhomboid or trapezoid. The wrap-sheet may have two end edges and two side edges. It may fully enwrap the structure such that the wrap-sheet is attached to the absorbent article (e.g. to the topsheet) with its garment-facing surface and the structure is attached to the absorbent article via its attachment to the wearer-facing surface of the wrap-sheet (such as by attaching the first layer of a so-called cell-forming structure described in detail below to the wearer-facing surface of the wrap-sheet). Alternatively, the wrap-sheet may be attached to the absorbent article, such as to the topsheet, at or adjacent to one of its end edges, extend over the longitudinal or lateral dimension of the structure and be attached to the absorbent article, such as to the topsheet, at or adjacent the respective other end edge. The wrap-sheet may be further attached to the absorbent article, such as to the topsheet, at or adjacent its side edges. The wrap-sheet may be fully or partly wrapped around the structure such that conversion of the structure from its flat configuration into its erected configuration is not obstructed.

To enable unobstructed conversion of the structure from its flat configuration into its erected configuration, the wrap-sheet has to be able to elongate. A suitable wrap-sheet may comprise slits. Upon erection of the structure, the slits in the wrap-sheet are able to open up and form apertures, and the wrap-sheet thus elongates. Alternatively, the wrap-sheet may be formed with folds. Upon erection of the structure, the folds in the wrap-sheet unfold, and the wrap-sheet elongates. In still another alternative, the wrap-sheet may be made of extensible material, such as extensible nonwoven. The material may also be rendered extensible, i.e. by incrementally stretching the material (so-called "ring-rolling").

However, it is less desirable that the wrap-sheet is made of elastic material. While such material can undergo elastic elongation while it is stretched due to erection of the structure, the wrap-sheet due to its tendency to retract, may exert a pressure onto the structure below the topsheet, which may lead to a decrease in caliper of the structure.

Generally, the dimension of the wrap-sheet can be considerably larger than the longitudinal and/or lateral dimension of the structure. Hence, the wrap-sheet may cover an area on the topsheet which is at least 1.5, or at least 1.8, or at least 2, or at least 3 or at least 4, or at least 5 times the topsheet area covered by the structure in its flat configuration. However, the wrap-sheet will generally be smaller than the topsheet and will not cover the complete surface area of the topsheet. It may cover less than 80%, or less than 60% or less than 50% or less than 40% of the surface of the topsheet.

Also, given that the wrap-sheet is positioned above the topsheet and often in areas where body liquid enters or spreads within the absorbent article, the wrap-sheet is desirably made of material pervious to body liquids (urine).

Alternatively or in addition to a wrap-sheet, the material forming the distal end of the erected structure (such as the second layer of a cell-forming structure as described below) may be larger than the other components of the structure, thus covering the other components of the erected structure. The material forming the distal end of the erected structure may be attached to the absorbent article (e.g. to the topsheet) in a manner as to not obstruct conversion of the structure from its flat configuration into its erected configuration (e.g. by having folds in the material forming the distal end of the erected structure which folds are present when the structure is in its flat configuration and flatten out upon erection of the structure).

The one or more elastic elements may be comprised by the wrap-sheet and be associated with (e.g. may be attached to) the structure via the wrap-sheet. Alternatively, the one or more elastic elements may be comprised by the structure and be associated with (e.g. attached to) the wrap-sheet. Still alternatively, the one or more elastic elements may be comprised by the structure, such as by the distal end of the structure, and the distal end of the structure is associated with (e.g. attached to) the wrap-sheet.

**General considerations for the structure irrespective whether placed below or above the topsheet**

The structure is positioned on or below the topsheet (but generally above the absorbent core) and often in areas where body liquid enters or spreads within the absorbent article. Thus, it is desirable that the structure is made of liquid pervious material. The material of the structure may also be hydrophilic.

The longitudinal dimension of the structure may be parallel or transverse to the longitudinal dimension of the absorbent article. Still alternatively, the longitudinal dimension of the structure may be arranged at an angle from the longitudinal and transversal axis of the absorbent article.

The longitudinal and lateral dimension of the structure inter alia depends on the size of the absorbent article: For example, in large absorbent articles for adult users, the absorbent article may generally have substantially larger longitudinal and lateral dimensions compared to absorbent articles for babies or toddlers. The longitudinal and lateral dimensions of the structure may also depend on the orientation of the structure within the absorbent article. Moreover, the longitudinal and lateral dimension of the structure may also depend on the function of the structure.

If the structure is placed below the topsheet and intended to lift the absorbent article (here: often an article to be worn by adults, and the lateral dimension of the structure is parallel with the transverse axis of the absorbent article, the lateral dimension of the structure may from 10 mm to 500 mm, or from 20 mm to 300 mm, or from 30 mm to 250 mm.

If the structure is placed below the topsheet and intended to lift the absorbent article (here: often an article to be worn by adults, and the lateral dimension of the structure is parallel with the longitudinal axis of the absorbent article, the lateral dimension of the structure may from 10 mm to 200 mm, or from 20 mm to 150 mm, or from 30 mm to 100 mm.

If the lateral dimension of the structure is parallel with the transverse axis of the absorbent article, it can also serve as a transverse barrier in the absorbent article which helps to reduce soiling of the genitals or other skin by the feces, or to reduce mixing of urine and feces, to further reduce the risk of irritation of the skin. In such cases, the lateral dimension of the structure may often be relatively small, such as from 5 mm to 30 mm, or from 5 mm to 20 mm, or from 10 mm to 20 mm. In such embodiments, the structure may be attached to the barrier leg cuffs.

If the structure is placed below the topsheet and intended to lift the absorbent article (here: often an article to be worn by adults, and the longitudinal dimension of the structure is parallel with the transverse axis of the absorbent article, the longitudinal dimension of the structure may from 10 mm to 200 mm, or from 20 mm to 150 mm, or from 30 mm to 100 mm

If the structure is placed below the topsheet and intended to lift the absorbent article (here: often an article to be worn by adults, and the longitudinal dimension of the structure is parallel with the longitudinal axis of the absorbent article, the longitudinal dimension of the structure may from 10 mm to 500 mm, or from 20 mm to 300 mm, or from 30 mm to 250 mm.

If the structure is a cell-forming structure as described below, the preferred longitudinal dimensions given above will relate to the dimension between the two outermost ligaments along the longitudinal dimension of the structure.

The longitudinal and lateral dimensions are determined when the structure is in its initial flat configuration and are determined by measuring the area of the distal end of the structure (if the structure is a cell-forming structure, the distal end of the structure is formed by the second layer).

The non-elastic materials comprised by the structure may have a bending stiffness of less than 500 mNm, preferably less than 400 mNm, more preferably less than 200 mNm.

The non-elastic materials comprised by the structure may have a tensile strength of less than 80 N/cm, preferably less than 50 N/cm, and more preferably less than 40 N/cm.

The structure is in a flat configuration when the absorbent article is flattened out and when the one or more elastic elements are stretched. Upon contraction of the one or more elastic elements, the structure is able to convert into an erected, three-dimensional configuration. The erected structure spaces one end (which thus becomes the distal end) of the structure away from the topsheet if the structure is placed onto the topsheet. If the structure is placed below the topsheet, the erected structure spaces one end (becoming the distal end) of the structure as well as the portion of the topsheet which is above the structure (and one or more layers of the acquisition system, if they overly the structure), away from underlying components of the absorbent article. The proximate end of the erected structure however, remains permanently attached to the absorbent article.

The increase in caliper of the erected structure vs. the flat structure may be more than 5 mm, or more than 8 mm, or more than 10 mm, or more than 15 mm, or more than 20 mm, or more than 25 mm, or more than 30 mm, or more than 40 mm. Also, the increase in caliper of the erected structure may be less than 100 mm, or less than 80 mm, or less than 60 mm, or less than 50 mm vs. the flat structure. Generally, the increase in caliper will depend on the intended use, with absorbent articles intended to be worn by babies and small toddlers requiring a smaller caliper whereas absorbent articles to be worn by larger children or adults may require a higher caliper. Also, for sanitary napkins or pantiliners or other absorbent inserts to be worn by placing it in the wearer's undergarment, a smaller caliper may be sufficient versus the use in a diaper or pant.

The structure may be positioned in the crotch region, in the first waist region (the front waist region lying against the belly of a wearer in use) or, though less preferred, in the second waist region (the back waist region lying against the back of the wearer in use). The structure may also extend into the crotch and first waist region (front waist region).

The one or more elastic elements may be comprised by or associated with the distal end of the structure, i.e. the end of the structure which is lifted upwardly towards the skin of the wearer when the absorbent article is in use.

**Cell forming structures**

Examples of structures suitable for use in absorbent articles of the present invention are so-called "cell forming structures".

The name "cell forming structures" is due to the cells formed between neighboring ligaments in the erected structure configuration, the cells being delimited by two neighboring ligaments and the first and second layer. These structures are initially relatively flat. When a force is applied along the longitudinal dimension (i.e. along the lengthwise extension) of the structure, the structure adopts an erected configuration. Thus, the structure increases in caliper. As used herein, the terms "caliper" and "thickness" are used interchangeably and refer to a direction perpendicular to the lateral and longitudinal dimension.

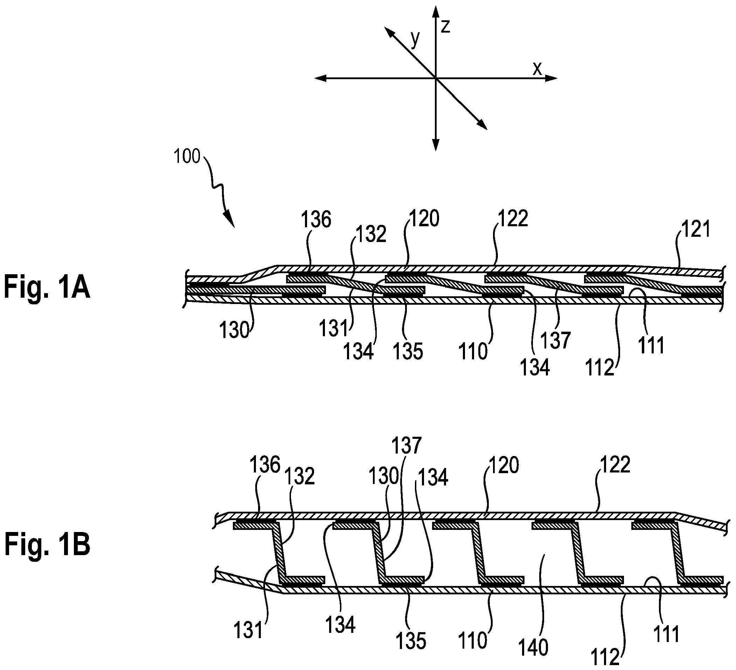

Figure 1A shows a cell-forming structure -with ligaments having Z-like shape- in its flat configuration whereas Figure 1B shows the structure in its erected configuration.

Generally, the structure (100) of the present invention comprises a first and a second layer (110, 120).

The first layer (110) may be attached to the component of the absorbent article, which is below the structure. Hence, the first layer may be attached to the topsheet (if the structure is placed above the topsheet), to the secondary topsheet, to a layer of the acquisition system or to the absorbent core. Alternatively, the first layer may be made of a portion of the component of the absorbent article, which is below the structure. Hence, the first layer may be made of a portion of the topsheet (if the structure is placed above the topsheet), of a portion of the secondary topsheet, of a portion of a layer of the acquisition system, or of a portion of a layer of the absorbent core (such as a nonwoven absorbent core cover). The first layer may be made of non-elastic or highly non-elastic material. Also the first layer may be made non-extensible or highly non-extensible material. The first layer may form the proximate end of the erected structure.

The second layer (120) may predominately be made of non-extensible or highly non-extensible material. However, the second layer may comprise the one or more elastic element of the structure, such that the second layer has elastic portions comprising the one or more elastic elements and non-elastic portions made of non-elastic material. Alternatively, the one or more elastic element may be directly or indirectly associated with the second layer (120) (such that the one or more elastic elements may be comprised by the topsheet, or by the wrap-sheet described above), in which case the second layer may be made of non-elastic material. The second layer may form the distal end of the erected structure.

The first and second layers may be made of nonwovens, film, paper, sheet-like foam, woven fabric, knitted fabric or combinations of these materials. Combinations of these materials may be laminates, e.g. a laminate of a film and a nonwoven. Generally, a laminate may consist of only two materials joined to each other in a face to face relationship and lying upon another but alternatively may also comprise more than two materials joined to each other in a face to face and lying upon another.

The one or more elastic elements which may be comprised by the second layer may be elastic film, elastic nonwoven, elastic scrim or elastic strands.

The first and second layer may be made of the same material (apart from the one or more elastic elements if they are comprised by the second layer). Alternatively, the first layer may be made of material which is different from the material of the second layer.

The first and second layer may generally be made of two separate pieces of material.

The (non-elastic) materials of the first and second layer may be chosen such that the first and second layers have the same basis weight, tensile strength, bending stiffness, liquid permeability, breathability and/or hydrophilicity. Alternatively, the first and second layer may differ from each other in one or more properties, such as basis weight, tensile strength, bending stiffness, liquid permeability, hydrophilicity/hydrophobicity and/or breathability.

The basis weight of the first and second layer may be at least 2 g/m$^2$, or at least 3 g/m$^2$, or at least 5 g/m$^2$; or at least 8 g/m$^2$, or at least 10 g/m$^2$, or at least 12 g/m$^2$, and the basis weight may further be not more than 500 g/m$^2$, or not more than 200 g/m$^2$, or not more than 100 g/m$^2$, or not more than 50 g/m$^2$, or not more than 30 g/m$^2$. If the second layer comprises the one or more elastic elements, the basis weight of the second layer, as used herein, refers to the non-elastic material of the second layer as well as to the one or more elastic elements.

The tensile strength of the first and second layer may be at least 3 N/cm, or at least 4 N/cm, or at least 5 N/cm. The tensile strength may be less than 100 N/cm, or less than 80 N/cm, or less than 50 N/cm, or less than 30 N/cm, or less than 20 N/cm. If the second layer comprises the one or more elastic elements, the tensile strength of the second layer refers to the non-elastic materials only.

The bending stiffness of the first and second layer may be at least 0.1 mNm, or at least 0.2 mNm, or at least 0.3 mNm. The bending stiffness may be less than 200 mNm, or less than 150 mNm, or less than 100 mNm, or less than 50 mNm, or less than 10 mNm, or less than 5 mNm. If the second layer comprises the one or more elastic elements, the bending stiffness of the second layer refers to the non-elastic materials only.

Generally, the higher the tensile strength and the bending stiffness of the first and second layer, the more rigid, but also the more stable the overall structure will become. Hence, the choice of tensile strength and bending stiffness for the first and second layer depends on the application of the structure, balancing overall softness, drape and conformability requirements with overall stability and robustness. The bending stiffness of the first and second layer may be chosen such as to ensure that the contracting forces of the one or more elastic elements do not result in excessive buckling of the structure, so that erection of the structure is not adversely impacted.

The first and second layers (110, 120) are connected to each other via ligaments (130).

In the structure (100), each of the first and second layers (110, 120) has an inner (111, 121) and an outer surface (112, 122) wherein the inner surfaces (111, 121) face towards the ligaments (130). Each of the first and second layer (110, 120) in the structure (100) further has a longitudinal dimension which is parallel to the longitudinal dimension of the structure (100) and which is confined by two spaced apart lateral edges. Each of the first and second layers (110, 120) also has a lateral dimension parallel with the lateral dimension of the structure and confined by two spaced apart longitudinal edges. The first and second layer (110, 120) in the structure (100) overlap at least in the area where the ligaments are positioned between the first and second layer. The first and second layer (110, 120) may also overlap -at least partly- in the areas extending outboard of the area where the ligaments (130) are positioned.

The ligaments (130) are positioned in between the first and second layer (110, 120). Each ligament (130) has a longitudinal dimension confined by two spaced apart lateral edges (134) and a lateral dimension confined by two spaced apart longitudinal edges.

In Figure 1, a coordinate system is shown with X-, Y- and Z-directions. The longitudinal dimension of the overall structure (100) and of the first and second layer (110, 120) extends along the longitudinal direction X of the coordinate system. The longitudinal dimension of the ligaments (130) in the structure's initial flat configuration may substantially extend along the longitudinal direction X of the illustrated coordinate system.

Likewise, the lateral dimension of the overall structure (100) and of the first and second layer(110, 120) extends along the lateral direction Y of the coordinate system. The lateral dimension of the ligaments (130) in the structure's initial flat configuration may substantially extend along the lateral direction Y of the illustrated coordinate system.

The caliper of the structure (100) extends along the Z direction of the coordinate system.

Each ligament (130) is attached to the inner surface (111) of the first layer (110) at or adjacent to one of the lateral edges (134) of the respective ligament. Each ligament (130) is also attached to the inner surface (121) of the second layer (120) at or adjacent to the other lateral edge (134) of the respective ligament. Due to this attachment, the ligaments (130) will generally adopt a C-like, Z-like, T-like, or Double-T-like shape when the structure is in its erected configuration.

When the ligament adopts a Z-like shape when the structure is in its erected configuration, the ligament (130) is attached to the inner surface (111) of the first layer (110) with a portion of the ligament's first surface (131) at or adjacent to one of its lateral ligament edges (134) in the first ligament attachment region (135) and is further attached to the inner surface (121) of the second layer (120) with a portion of the ligament's second surface (132) at or adjacent to its other lateral ligament edge (134) in the second ligament attachment region (136).

Moreover, for Z-like shapes, the ligament's (130) first surface (131) may not be facing towards the inner surface (121) of the second layer (120) when the structure (100) is in its initial flat configuration, and the ligament's second surface (132) may not be facing towards the inner surface (111) of the first layer (110) when the structure (100) is in its initial flat configuration. With such embodiments, it is possible to obtain structures with no folds and hinges (or very few folds and hinges, e.g. when the ligaments have different longitudinal dimension in their free intermediate portion (137), see below). Hence, these structures will generally exhibit a lower tendency to partly erect in the absence of an applied force along the longitudinal dimension and will consequently remain in their initial flat configuration. Also, such structures will generally exhibit a greater tendency to return to their initial flat configuration when the applied force is released.

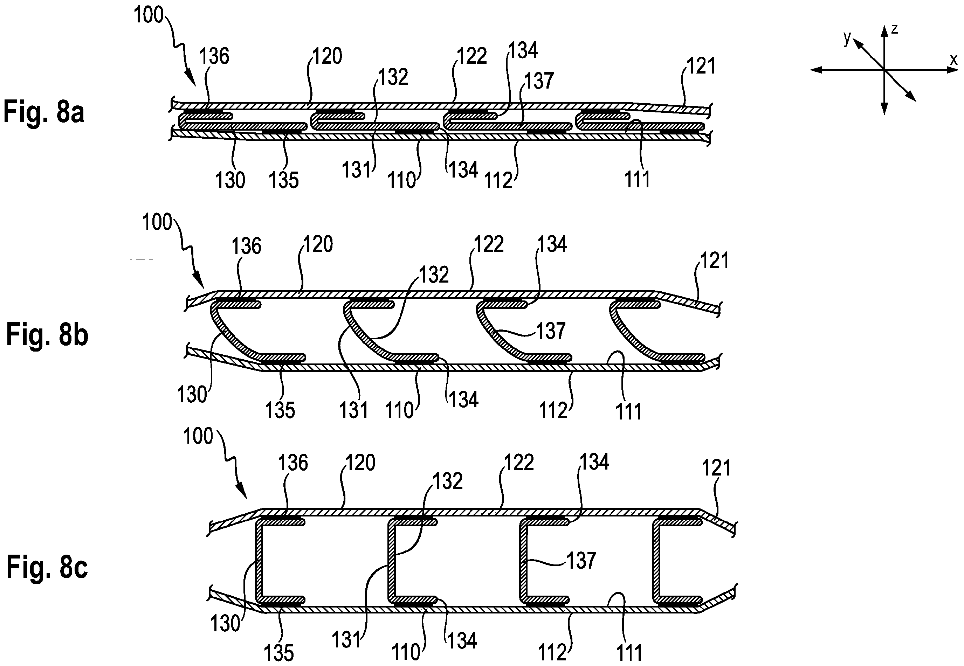

Alternatively, when the ligament adopts a C-like shape when the structure is in its erected configuration (as exemplary shown in Fig. 8A through 8C), the ligament (130) is attached to the inner surface (111) of the first layer (110) with a portion of the ligament's first surface (131) at or adjacent to one of its lateral ligament edges (134) in the first ligament attachment region (135) and is further attached to the inner surface (121) of the second layer (120) with a portion of the ligament's first surface (132) at or adjacent to its other lateral ligament edge (134) in the second ligament attachment region (136).

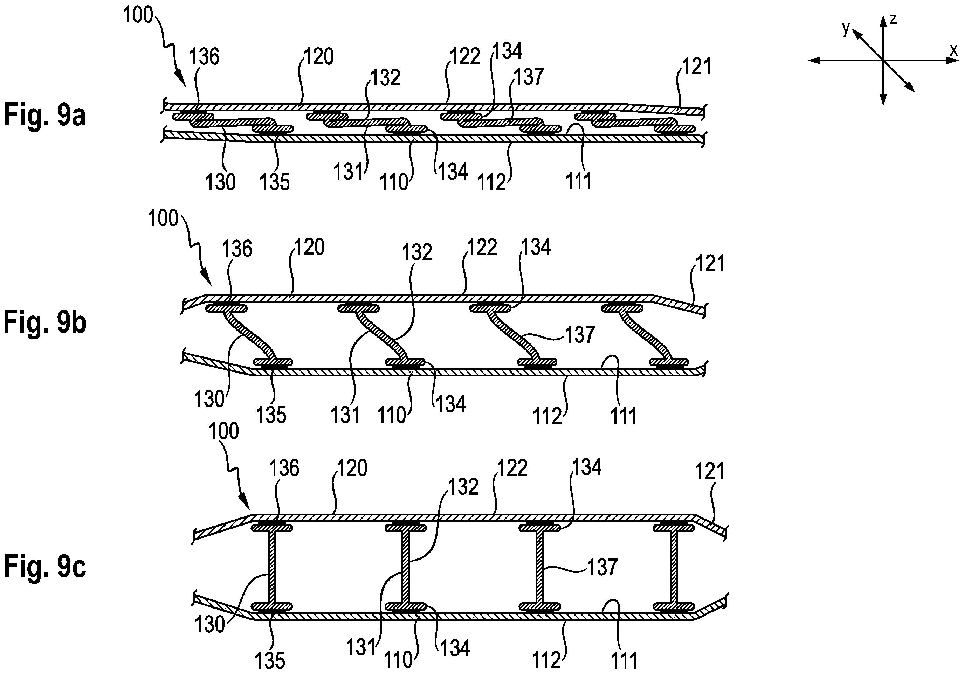

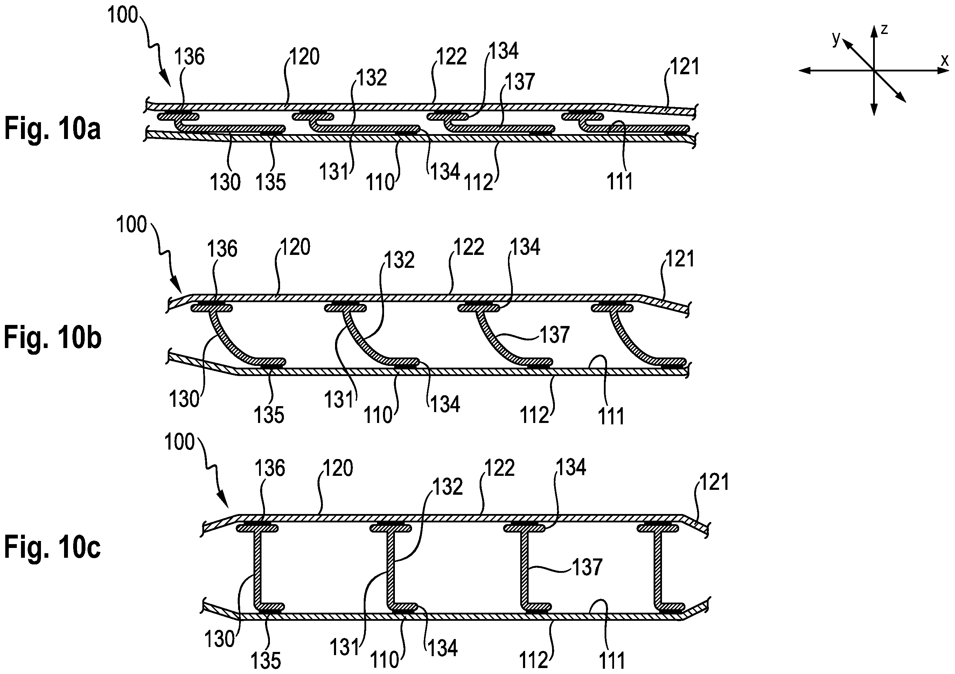

Still alternatively, one portion of the ligament at or adjacent to one of its lateral ligament edges may be attached to the first layer such as to form a T-like (as exemplary shown in Fig. 10A through 10C), or Double-T-like shape (as exemplary shown in Fig. 9A through 9C). To facilitate such attachment, the portion of the ligament adjacent to a first lateral ligament edge has to comprise at least two ligament layers and the two ligament layers are not attached to each other in the portion of the ligament which is attached to the first layer. Thereby, the ligament can be split up and unfolded such that both ligament layers can be respectively attached to the first layer in the first ligament attachment region (135) to form a T-like shape when the structure is in its erected configuration.

For embodiments where a ligament adopts a T-like shape, the portion of the ligament at or adjacent to the second lateral ligament edge is attached to the second layer with either its first or second surface to form the second ligament attachment region (136). If the ligament is also made of a laminate in the second ligament attachment region (136), the ligament layers are not split up in this area (i.e. only the portion adjacent to the first lateral ligament edge forms a T-like shape).

When the ligament adopts a Double-T-like shape when the structure is in its erected configuration, the portion of the ligament at or adjacent to the second lateral ligament edge is attached to the second layer similar to the manner in which the portion at or adjacent to the first lateral ligament edge is attached to the first layer, i.e. the ligament laminate is split up and unfolded such that two ligament layers can be respectively attached to the second layer to form the second ligament attachment region (136).

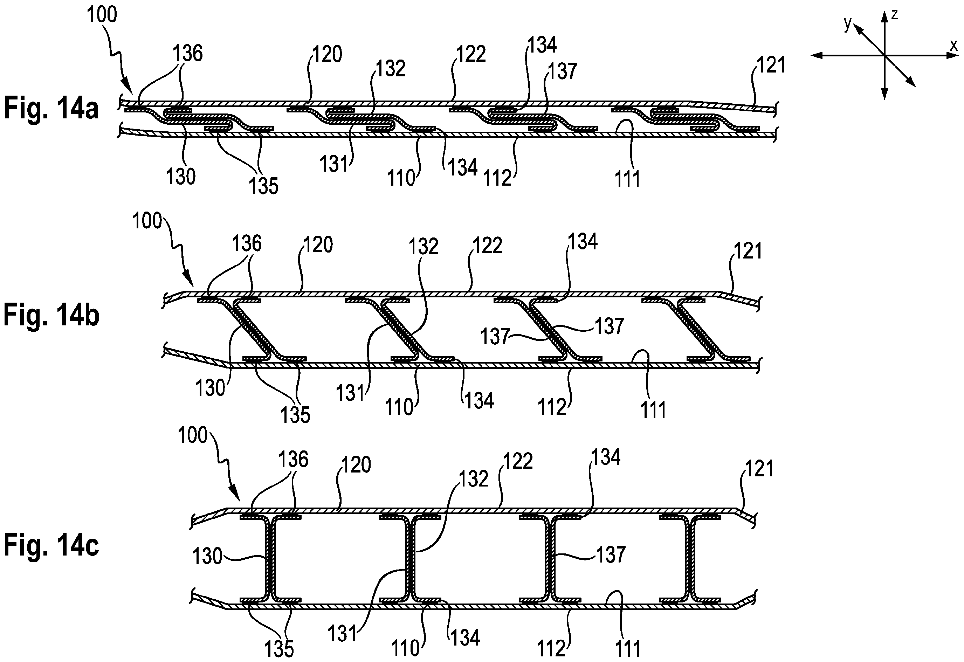

Splitting up and unfolding the ligament layers to form the respective first and second ligament attachment regions (135, 136) is exemplified in Figures 14A through 14C (showing ligaments with Double-T-like shape).

In a given structure, all ligaments may adopt a Z-like shape when the structure is in its erected configuration. Alternatively, all ligaments may adopt a C-like shape, all ligaments may adopt a T-like shape or all ligaments may adopt a Double-T-like shape when the structure is in its erected configuration. In a further alternative, a given structure may have a combination of ligaments selected from the group consisting of: ligaments adopting a Z-like shape, C-like shape, T-like shape and Double-T-like shapes.

In a given structure, ligaments adopting a Z-like shape when the structure is in its erected configuration may all adopt the same orientation. That means, in such embodiments none of the ligaments adopting a Z-like shape has an inverse configuration of another ligament adopting a Z-like shape.

Likewise, in a given structure, ligaments adopting a C-like shape when the structure is in its erected configuration may all adopt the same orientation. That means, in such embodiments none of the ligaments adopting a C-like shape has an inverse configuration of another ligament adopting a C-like shape.

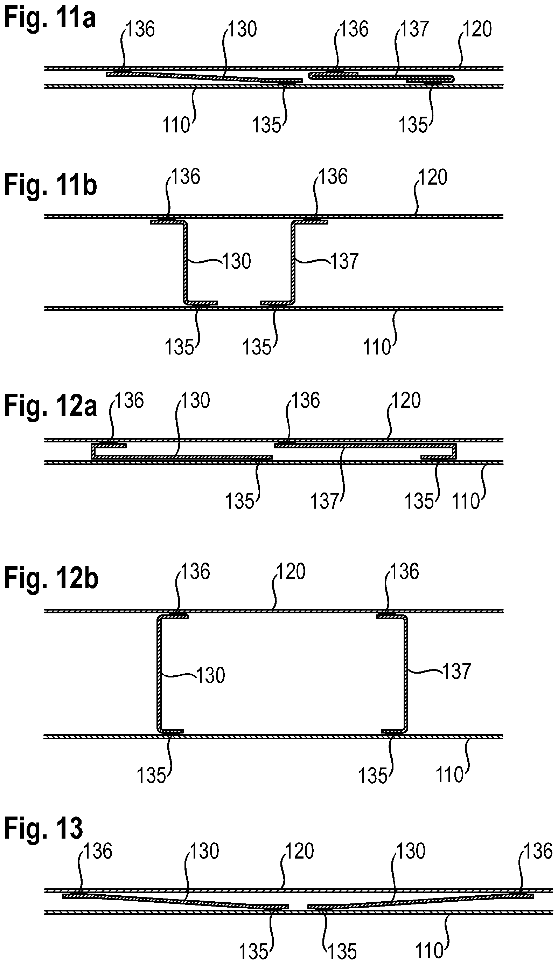

However, it is also possible to facilitate the structure such that that one or more ligaments adopt a Z-like shape which has an inverse configuration of one or more other ligaments with Z-like shape. An example of such structure is illustrated in Fig. 11A (flat configuration) and Fig. 11B (erected configuration).

Similarly, a structure with one or more ligaments adopting a C-like shape which has an inverse configuration of one or more other ligaments with C-like shape is exemplified in Fig. 12A (flat configuration) and Fig.12B (erected configuration).

Such inverse configurations are feasible as long as the structure can be readily converted from its flat configuration to its erected configuration. An example of a structure having Z-like ligaments with inverse configuration which can only be converted from its flat to erected configuration when the one or more elastic elements are provided in the second layer (120) between the two neighboring ligament attachment regions (136) shown in Fig.13. If no elastic element(s) are provided between the two neighboring ligaments, the ligaments on the right side of the Figure will "block" the ligaments on the left side of the Figure from being erected upon release of the contractive force, which has provided by the stretched one or more elastic elements along the longitudinal direction (and vice versa, the ligaments on the left side of the Figure "block" the ligaments on the left side of the Figure from being erected). Generally, the ligaments shown on the right side (or left side) of the Figure also cannot serve as a stop aid (explained below in detail) as no leeway is provided.

Generally, ligaments in a given structure have to be configured and attached accordingly such that the structure is able to be converted from an initial flat configuration into an erected configuration whereby the ligaments convert from an initial flat configuration into an erected configuration. Moreover, the ligaments in a given structure have to be configured and attached accordingly such that, upon further release of the contractive force, which has provided by the stretched one or more elastic elements along the longitudinal dimension, it would be possible -in the absence of a means that maintains the structure in its erected configuration, such as a stop aid, which is described below- to convert the erected structure into a turned-over flat structure, wherein the ligaments would have been turned over by 180° based on the ligament's position in the initial flat structure configuration.

The longitudinal dimension of each ligament (130) between the first and second ligament attachment regions (135, 136) remains unattached to the first and second layer (110, 120) or is releasable attached to the first and/or second layers (110, 120) and/or to their neighboring ligament(s). This unattached or releasable attached portion is referred to as the "free intermediate portion" (137) of the ligament (130). "Releasable attachment of ligaments" means a temporary attachment to the first and/or second layer (110, 120) and/or the neighboring ligament(s) in a way, that the bond strength is sufficiently weak to allow easy detachment from the first and/or second layer and/or the neighboring ligament(s) upon initial contraction of the structure (100) along the longitudinal dimension without rupturing or otherwise substantially damaging the ligaments (130) and/or the first and/or second layer (110, 120) and without substantially hindering the conversion of the structure from its initial flat configuration into its erected configuration. Such releasable attachments may help to maintain the structure in its initial flat configuration, e.g. during manufacturing processes when the structure is incorporated into an article.

When the structure is in its initial flat configuration, the first surface (131) of a ligament's free intermediate portion (137) faces towards the first layer (110) and the second surface (132) of a ligament's free intermediate portion (137) faces towards the second layer (120). When the structure (100) is converted into its erected configuration, the first surface (131) of a ligament's (130) free intermediate portion (137) faces towards the second surface (132) of its neighboring ligament (130). This is irrespective as to whether the ligament has been attached to adopt a Z-like, C-like, T-like or Double-T-like shape. Unless expressly mentioned herein, neighboring ligaments refers to ligaments which are neighboring along the longitudinal dimension.

The ligaments (130) may be attached to the first and second layer (110, 120) by any means known in the art, such as by use of adhesive, by thermal bonding, by mechanical bonding (such as pressure bonding), by ultrasonic bonding, or by combinations thereof. The attachment of the ligaments to the first and second layer is permanent, i.e. the attachment should not be releasable by forces which can typically be expected during use of the structure.

Structures (100) as described supra are able to adopt an initial flat configuration when no external forces are applied. Upon release of the contractive force, which has provided by the stretched one or more elastic elements along the longitudinal dimension, the structure will increase in caliper, i.e. in the direction perpendicular to the longitudinal and lateral dimension.

The force along the longitudinal dimension may be applied by appropriately positioning the one or more elastic elements. For example, one or more elastic element (196) may be comprised by and/or attached to the second layer longitudinally outboard of the second ligament attachment region (136) which is closest to one of the lateral edges of the structure. The second layer (120) may be permanently attached (197) to the first layer (110), to the topsheet (if the structure is placed on top of the topsheet), to the secondary topsheet, to the acquisition system and/or to the absorbent core longitudinally outboard of the one or more elastic elements or may be permanently attached to the first layer (110 to the topsheet (if the structure is placed on top of the topsheet), to the secondary topsheet, to the acquisition system and/or to the absorbent core with the elastic material comprised by the second layer. The one or more elastic elements may be comprised by or associated with the second layer only in the area longitudinally outboard of the second ligament attachment region (136) which is closest to one of the lateral edges of the structure. Alternatively, the one or more elastic elements may be continuously comprised by or be associated with the second layer from the area longitudinally outboard of the second ligament attachment region (136) which is closest to one of the lateral edges of the structure into the area between neighboring second ligament attachment regions (136) (such that in the erected structure, the one or more elastic element is also comprised by or associated with the second layer between neighboring ligaments). Still alternatively, the one or more elastic elements may be comprised by or be associated with the second layer continuously along the complete longitudinal dimension of the second layer. The second layer may also be completely formed by the elastic element.

When the structure is in its flat configuration, the one or more elastic elements are in a stretched condition. On the respective other side of the one or more elastic elements along the longitudinal dimension of the structure, e.g. at or adjacent to the respective other lateral edge of the structure, the second layer (120) may be releasably attached (198) to the first layer (110), to the topsheet (if the structure is placed on top of the topsheet), to the secondary topsheet, to the acquisition system and/or to the absorbent core. If the one or more elastic element extends over the complete longitudinal dimension of the second layer, the releasable attachment (198) may also encompass the one or more elastic element, i.e. the releasable attachment is between the one or more elastic element of the second layer and the first layer(110 the topsheet (if the structure is placed on top of the topsheet), the secondary topsheet, the acquisition system and/or the absorbent core.

The one or more elastic element may take the form or one or more elastic strands comprised by the second layer. Alternatively, the one or more elastic elements may be sheet-like materials, such as elastic film or elastic nonwoven, which is comprised by the second layer.

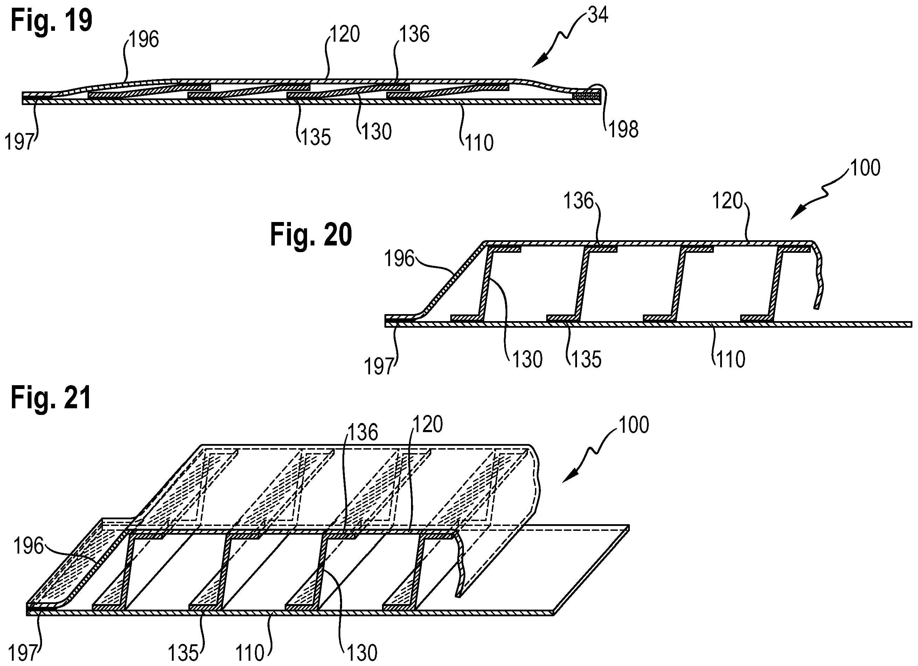

Upon release of the releasable attachment, the one or more elastic element contracts, thus applying a force along the longitudinal dimension of the structure. Thereby, the free intermediate portion of the ligaments lifts upward away from the first layer (and away from the second layer), thus lifting the second layer upwardly and converting the structure into its erected configuration. An example of such a structure is shown in Figures 19-21.

Alternatively or in addition to providing or associating the one or more elastic element in the second layer and/or attaching one or more elastic element to the second layer longitudinally outboard of the ligament which is closest to one of the lateral edges of the structure, one or more elastic element (195) may be comprised by the second layer (120) in an area between two neighboring ligaments (i.e. between neighboring second ligament attachment regions), and may not be comprised by or associated with the second outermost layer in the areas longitudinally outboard of the second ligament attachment region (136) which is closest to one of the lateral edges of the structure. Likewise to the above, these one or more elastic elements (195) are stretched when the structure is in its flat configuration. Also similar to the above, the structure is maintained in its initial flat configuration due to a releasable attachment (198) of the second layer (120) to the first layer (110), to the topsheet (if the structure is placed on top of the topsheet), to the secondary topsheet, to the acquisition system and/or to the absorbent core. However, compared to the configuration described in the previous paragraph, here the second layer is releasably attached to the first layer, to the topsheet (if the structure is placed on top of the topsheet), to the secondary topsheet, to the acquisition system and/or to the absorbent core on both sides outside of the one or more elastic elements (viewed along the longitudinal dimension of the structure). There may hence not be a permanent attachment of the second layer to the first layer, to the topsheet (if the structure is placed on top of the topsheet), to the secondary topsheet, to the acquisition system and/or to the absorbent core. Upon release of these releasable attachments (198), the one or more elastic element (195) comprised by the second layer (120) between two neighboring ligaments contracts, thus applying a force along the longitudinal dimension of the structure.

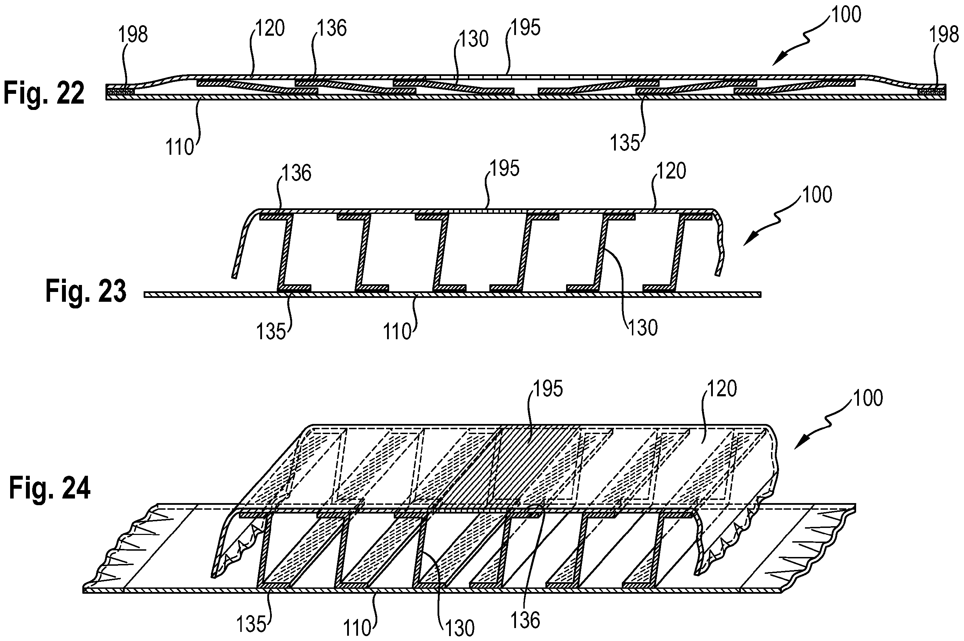

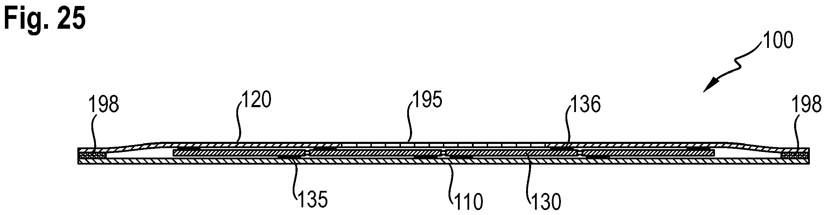

Other than in the above embodiment, where the force is applied in the same direction along the longitudinal dimension of the structure along the complete structure, here the forces are applied in opposite directions along the longitudinal dimension of the structure: I.e. for the areas of the structure on one side (along the longitudinal dimension) of the one or more elastic elements the force is applied towards the one or more elastic element and for the areas of the structure on the respective other side (along the longitudinal dimension) of the one or more elastic elements, the force is applied towards the one or more elastic element and hence, in the opposite direction vs. the area on the other side of the one or more elastic elements. Examples for such structures are shown in Figures 22 through 26.

It is possible to provide one or more elastic elements between neighboring second ligament attachment regions for more than two neighboring second ligament attachment regions, e.g. by providing one or more elastic element between two neighboring second ligament attachment regions and providing further elastic element(s) between two different neighboring second ligament attachment regions. Likewise, several elastic elements can be provided between a number of neighboring second ligament attachment regions.

The one or more elastic elements, especially the dimension of the one or more elastic elements, should to be facilitated accordingly, such that upon full contraction of the elastic element(s), the free intermediate portions of the ligaments are properly lifted and the overall structure is transferred into its erected configuration. The exact configuration of the one or more elastic elements thus need to be adapted to the respective overall design of the structure, taking into consideration aspects such as longitudinal dimension of the ligament's free intermediate portions, the distance between neighboring ligaments, the dimension of the one or more elastic elements in their contracted state etc.

Also, the orientation of the ligaments needs to be configured accordingly to enable proper erection of the ligaments upon contraction of the one or more elastic elements. Examples are shown in Figures 19 through 26.

By permanently attaching the second layer to the first layer, to the topsheet (if the structure is placed on top of the topsheet), to the secondary topsheet, to the acquisition system and/or to the absorbent core, the one or more elastic elements can be coupled to the rest of the absorbent article, which helps bending of the absorbent article such that, if desired, the absorbent article adopts an overall arcuate form upon contraction of the one or more elastic elements of the structure.

In the structure described above, where the second layer is releasably attached to the first layer, the topsheet (if the structure is placed on top of the topsheet), the secondary topsheet, the acquisition system and/or the absorbent core, on both sides (along the longitudinal dimension of the structure) of the one or more elastic elements, the second layer may be permanently attached to the first layer, to the topsheet (if the structure is placed on top of the topsheet), to the secondary topsheet, to the acquisition system and/or to the absorbent core indirectly via a layer-to-layer or a layer-to-ligament stop aid as described below in detail, in order to couple the one or more elastic elements to the rest of the absorbent article, and help bending of the absorbent article, if desired, such that the absorbent article adopts an overall arcuate form upon contraction of the one or more elastic elements of the structure. Alternatively or in addition, the second layer may be permanently attached to the first layer, to the topsheet (if the structure is placed on top of the topsheet), to the secondary topsheet, to the acquisition system and/or to the absorbent core longitudinally outboard of the releasable attachments. In such cases, a leeway has to be provided in the second layer between the releasable attachment and the permanent attachment. Upon release of the releasable attachment, the one or more elastic elements contract and the structure erects. The leeway flattens out (or extends, in case the leeway is provided by extensible or elastic material - which is in a non-stretched state when the structure is in the flat configuration similar to the provision of the stop aid, see below), which can induce an overall arcuate form of the absorbent article.

Unless specifically mentioned herein, "attached" or "attachment" means directly attached.

It is also possible to combine one or more elastic element which is associated with the second layer with one or more elastic elements which are comprised by the second layer.

The ligaments must be attached appropriately for each of the described structures to enable erection of the structure upon contraction of the one or more elastic elements. I.e. the ligaments should not be attached such as to hinder proper erection (see also above with respect to Fig.13).

The structure can be facilitated such that, in its initial flat configuration, no hinges and folds may be created in the structure (100) and the first and second layers (110, 120) as well as the ligaments (130) lie flat and outstretched. Such structures allow having a very thin caliper in the flat configuration. In these kinds of structures, all ligaments will adopt a Z-like shape when the structure is in its erected configuration.

In structures where no hinges may be created, the complete first surface (131) of each ligament (130) (i.e. not only the free intermediate portion) does not face towards the inner surface (121) of the second layer (110) when the structure (100) is in its initial flat configuration, and the complete second surface (132) of each ligament (130) (i.e. not only the free intermediate portion) does not face towards the inner surface (111) of the first layer (120) when the structure is in its initial flat configuration. Such a structure is illustrated in Fig. 1.

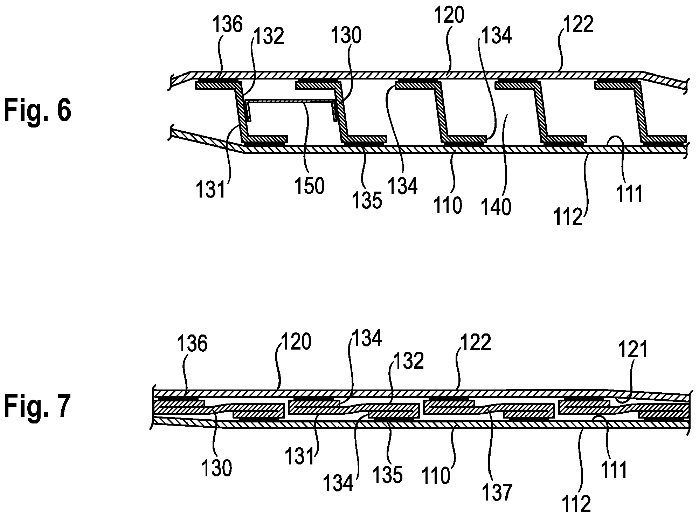

Figs. 7 and 8 show structures where the ligaments are folded when the structure is in its initial flat configuration.

Also, for certain other executions, e.g. those where the longitudinal dimension of the free intermediate portion (137) of the ligaments (130) differs from each other, the initial flat configuration may have some folds and hinges.

Upon erection of the structure (100) due to the erection of the ligaments (130), a space, a so-called "cell" (140) is formed between neighboring ligaments, which is confined by the first and second layer and the respective neighboring ligaments. When viewed from the side, along the lateral direction, the cells may take for example a rectangular shape, a trapezoid shape, a rhomboid shape, or the like. However, for use in absorbent articles according to the present invention, the structure will typically not form any rectangular shapes as the absorbent article will tend to flex upwardly along the longitudinal axis of the article, thus forming an overall arcuate form. The structure, being attached to the absorbent article, will follow this arcuate shape, and the shape of the cells will not be completely rectangular accordingly.

For structures wherein the longitudinal dimension of the free intermediate portion (137) is the same for all ligaments, the structure (100) will adopt its highest possible caliper when the ligaments (130) are in an upright position, i.e. when the free intermediate portion (137) of the ligaments (130) is perpendicular to the first and second layers (110, 120) between neighboring ligaments. However, the formation of this upright position may possibly be hindered, at least in some areas, when a force towards the caliper of the structure is applied at the same time, if this force is sufficiently high to deform the structure in the caliper dimension. The formation of such fully upright position may also be hindered when the absorbent article flexes and takes an arcuate form due to erection of the structure.

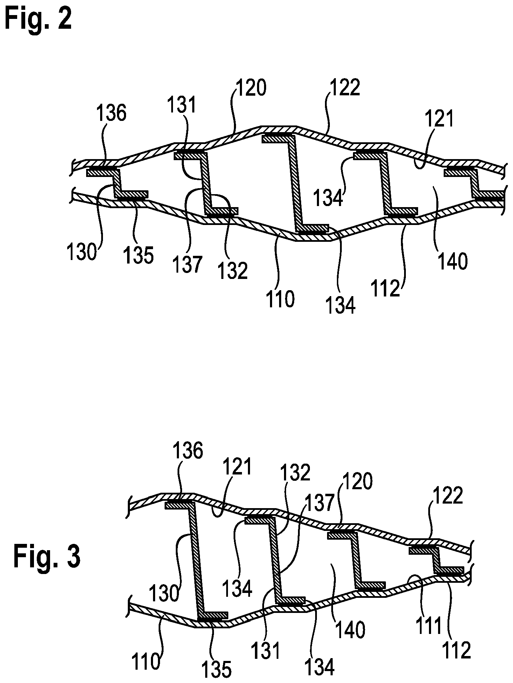

In structures, where the longitudinal dimension of the free intermediate portion (137) differs between different ligaments (130), the ligaments (130) may not be perpendicular to the first and second layer (110, 120) in the erected configuration, see e.g. Fig. 2 and 3). In such embodiments, the first and second layer (110, 120) are not parallel to each other when the structure is in its erected configuration but instead, the first and/or second layer (110, 120) take(s) an inclined shape. This may also happen due to the arcuate form of the absorbent article described above.

Tensile strength, and especially bending stiffness, impacts the resistance of the structure (especially of the structure in its erected configuration) against compression forces. Thus, when the ligaments have a relatively high tensile strength and bending stiffness, the structure is more resistant to forces applied in the Z-direction (i.e. towards the caliper of the structure). Also, if the first and/or second layers have relatively high tensile strength and relatively high bending stiffness, resistance of the structure against forces applied in the Z-direction (i.e. towards the caliper of the structure) is increased. For the present invention, the compression resistance of the erected structure is measured in terms of the structure's modulus according to the test method set out below.

As a difference in bending stiffness results in a difference in the resistance against compression forces (and hence, the modulus) applied in the Z-direction (i.e. towards the caliper of the structure), using ligaments with different bending stiffness enables structures which have improved resistance to compression (higher modulus) in the Z-direction in areas where the ligaments have higher bending stiffness, whereas the structure adjusts more readily e.g. to curved surfaces in the areas where ligaments with lower bending stiffness are applied (lower structure modulus). For example, the bending stiffness of the ligaments which are arranged in the center of the structure along the longitudinal dimension may be higher compared to the ligaments arranged towards the lateral edges of the structure.

In addition to the tensile strength and the bending stiffness of the materials used for the structure, the resistance of the (erected) structure against compression forces is also impacted by the number of ligaments which are provided, and the distance between neighboring ligaments. Neighboring ligaments which have a relatively small gap between them along the longitudinal dimension of the structure will provide for higher resistance of the erected structure against compression forces (higher modulus) compared to a structure wherein neighboring ligaments are more widely spaced apart along the longitudinal dimension of the structure (lower modulus) as long as the material used for the different ligaments and their size does not differ from each other.

Moreover, if the modulus is measured between neighboring ligaments, the modulus will typically be lower than the modulus measured in the location where a ligament is positioned.

Modulus is measured following the test method set out below and is measured in the Z-direction of the structure. Generally, the modulus of a structure is not measured while the structure is built into the absorbent article by ideally, prior to incorporating the structure into the absorbent article (otherwise, the structure needs to be carefully removed from the absorbent article).

The structure in its erected configuration may have a modulus of at least 0.004 N/mm$^2$, or at least 0.01 N/mm$^2$, or at least 0.02 N/mm$^2$, or at least 0.03 N/mm$^2$ in those areas where a ligament is positioned as well as in the areas between neighboring ligaments.

Moreover, it may also be desirable to avoid excessively high compression resistance (i.e. too high modulus), e.g. to avoid that the erected structure is too stiff, thus possibly leading to red marking on the wearer's skin in the area of the structure. For such structures, the structure in its erected configuration may have a modulus of not more than 1.0 N/mm$^2$, or not more than 0.5 N/mm$^2$, or not more than 0.2 N/mm$^2$, but at least 0.1 N/mm$^2$, or at least 0.5 N/mm$^2$, or at least 1.0 N/mm$^2$ in those areas where a ligament is positioned as well as in the areas between neighboring ligaments.

Alternatively, the structure in its erected configuration may have a modulus of at least 0.05 N/mm$^2$, or at least 0.08 N/mm$^2$, or at least 0.1 N/mm$^2$, or at least 0.13 N/mm$^2$, but not more than 2.0 N/mm$^2$, or not more than 1.0 N/mm$^2$, or not more than 0.5 N/mm$^2$, or not more than 0.3 N/mm$^2$ in the locations where the ligaments are positioned, while the structure in its erected configuration may have a modulus of at least 0.004 N/mm$^2$, or at least 0.01 N/mm$^2$, or at least 0.02 N/mm$^2$, or at least 0.03 N/mm$^2$ between neighboring ligaments.

Generally, the ligaments (130) may be spaced apart from each other along the longitudinal dimension at equal distances or, alternatively, at varying distances. Also, the ligaments (130) may be spaced apart from each other such, that the ligaments (130) do not overlap with each other when the structure (100) is in its initial flat configuration. Thereby, it is possible to provide structures (100) with very small caliper when the structure (100) is in its initial flat configuration, as the ligaments (130) do not "pile up" one on top of each other when the structure is in its initial flat configuration.

The free intermediate portion (137) of the ligaments (130) may all have the same longitudinal dimension. Thereby, the structure will have a constant caliper across its longitudinal (and lateral) dimension between neighboring ligaments when the structure (100) is in its erected configuration. An example of such an embodiment is shown in Figure 1B. Alternatively, the longitudinal dimension of the free intermediate portion (137) may vary for different ligaments (130) in a structure (100). Thereby, the caliper of the structure will vary across the longitudinal dimension. An example of such an embodiment is shown in Fig. 2 and 3.

As exemplified in Fig. 2, the one or more ligaments (130) in the center of the structure (as seen along the longitudinal dimension) may have a longer free intermediate portion (137) than the ligaments towards the lateral edges of the structure, resulting in a structure with a higher caliper in the center than towards the edges when the structure is in its erected configuration. Alternatively, one or more ligaments (130) towards one of the lateral edges may have a longer free intermediate portion (137) than one or more ligaments towards the other lateral edge, resulting in a structure with a wedge-like shape (when viewed from the side) when the structure is in its erected configuration. An example of such an embodiment is illustrated in Fig. 3. Generally, the caliper of the erected structure depends on the length of the free intermediate portion (137) of the ligaments (130).

Generally, the maximum increase in caliper of the structure is defined by the longitudinal dimension of the free intermediate portion (137) of the ligaments (130). If the ligaments (130) differ from each other in the longitudinal dimension of their free intermediate portions (137), the maximum increase in caliper of the structure in its erected configuration, as used herein, is defined by the longitudinal dimension of the ligament with the largest longitudinal dimension of free intermediate portion. If a stop aid (180, 190) is used (as described below), the structure may not be able to adopt its maximum increase in caliper in its erected configuration as the erection is stopped by the stop aid before the maximum erection, which would have been possible in the absence of a stop aid, is reached.

If formation of wrinkles in the first and second layer (110, 120) and/or in the ligament (130) shall be avoided when the structure is in its erected configuration, it is desirable, that the ligaments (130) are arranged such that, when the structure is in its initial flat configuration, the longitudinal dimension of the ligaments is substantially parallel with the longitudinal dimension of the first and second layer. "Substantially parallel" means that the orientation of the longitudinal dimension of the ligaments does not deviate by more than 20°, or not more than 10°, or not more than 5°, or not more than 2° from the longitudinal dimensions of the first and second layer. The orientation of the longitudinal dimension of the ligaments may also not deviate at all from the longitudinal dimension of the first and second layer.

Typically, the free intermediate portions (137) are not attached to each other. However, for certain applications it may be desirable that the free intermediate portion (137) of neighboring ligaments (130) are attached to each other directly, which results in some buckling of the ligaments attached to each other when the structure is in its erected configuration. Alternatively, the free intermediate portion (137) of neighboring ligaments (130) may be attached to each other indirectly via a separate ligament-to-ligament material (150), such as a piece of nonwoven, film, paper, or the like (shown in Fig. 6). If neighboring ligaments are attached to each other, especially via a separate piece of material, the overall stability and stiffness of the structure may be improved. This may be desirable in structures having relatively large caliper when the structure is in its erected configuration, as the free intermediate portion of the ligaments will have a relatively large longitudinal dimension. Also, in such embodiments, the cells formed between neighboring ligaments when the structure is in its erected configuration, are separated into sub-cells.

Generally, the first and second layer (110, 120) may have the same lateral dimension and the longitudinal edges of the first and second layer may be congruent with each other. Also, the lateral dimension of all ligaments (130) may be the same, and the lateral dimension of all ligaments (130) may also be the same as the lateral dimension of the first and second layer (110, 120). The longitudinal edge of the first layer (110) may coincide with the longitudinal edge of the second layer (120) and/or with the longitudinal edges of the ligaments (130).

Also, one or more of the ligaments (130) may have the same lateral dimension as the first and second layer (110, 120) and one or more of the ligaments, which do not have the same lateral dimension as the first and second layer may be flanked on one or both longitudinal edges by other ligaments, such that the structure has laterally neighboring ligaments.

## Ligaments

The ligaments may be elastic, but it is desirable that the ligaments are non-elastic or highly non-elastic. Also, the ligaments may be extensible, but it is desirable that the ligaments are non-extensible or highly non-extensible. The ligaments may be made of nonwovens, film, paper, or sheet-like foam, woven fabric, knitted fabric, or combinations of these materials. Combinations of these materials may be laminates, e.g. a laminate of a film and a nonwoven.

The ligaments within a structure may all be made of the same material or, alternatively, the different ligaments within a structure may be made of different materials.

The material may be the same throughout each ligament, i.e. the ligament may be made of a single piece of material or of a laminate wherein each laminate layer extends over the complete ligament.

The ligaments may have the same properties throughout the ligament, especially with regard to bending stiffness and tensile strength.

Alternatively, the ligaments may have areas with properties (such as bending stiffness and/or tensile strength) which differ from the properties in one or more other areas of a given ligament.

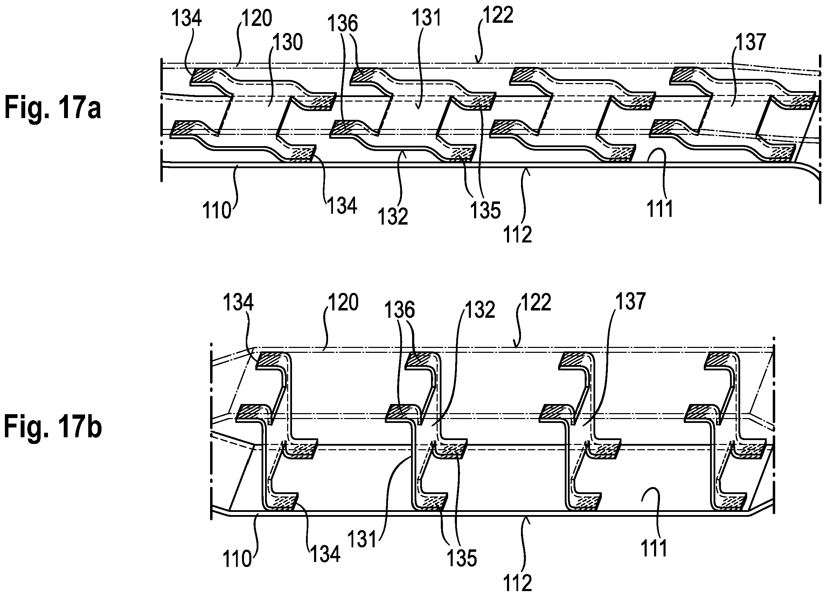

Such areas with differing properties can be facilitated by modifying the material in one or more ligament areas, e.g. by mechanical modification. Non-limiting examples of mechanical modifications are the provision of cut outs in one or more areas of the ligament to reduce tensile strength and bending stiffness in those areas; incremental stretching (so-called "ring-rolling") one or more ligament areas to reduce tensile strength and bending stiffness; slitting one or more ligament areas to reduce tensile strength and bending stiffness; applying pressure and/or heat to one or more areas of ligaments; or combinations of such mechanical modifications. Application of heat and/or pressure may either increase or reduce tensile strength and bending stiffness: For example, if heat and/or pressure are applied on a ligament made of nonwoven with fibers made of thermoplastic material, the fibers may be molten together and bending stiffness and tensile strength can be increased. However, if an excessive amount of heat and/or pressure is used, the material of the ligaments may be damaged (such as fiber breakage in a nonwoven web) and weakened areas are formed, thus reducing bending stiffness and tensile strength. Cutting out areas of the ligaments may either result in the formation of apertures within the ligament or the cut out may not be fully surrounded by uncut areas as is illustrated in Figures 17A (essentially flat configuration) and 17B (erected configuration).

Alternatively, or in addition to the above, areas with different properties can also be obtained by chemically modifying one or more areas of the ligament, e.g. by adding chemical compounds, such as binders or thermoplastic compositions to increase bending stiffness and tensile strength, which may be followed by curing. One or more areas with different properties can also be obtained by providing different materials in different areas or by adding additional pieces of materials only in certain areas (thus, e.g. forming a laminate in these areas), while having another material (which may be the same or different from the piece of material added in certain areas) which is coextensive with the ligament and used throughout the ligament.

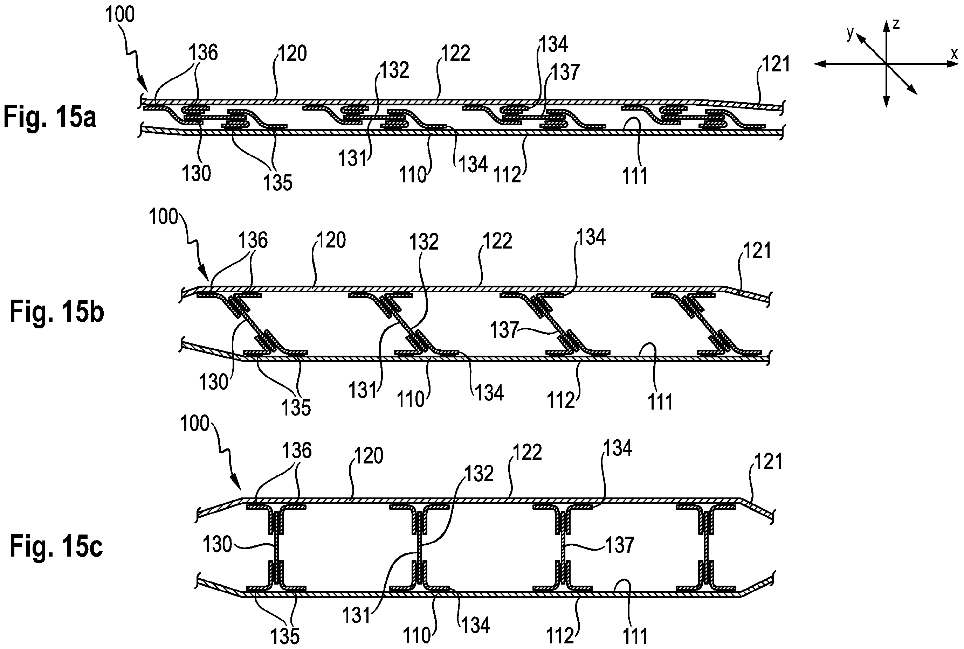

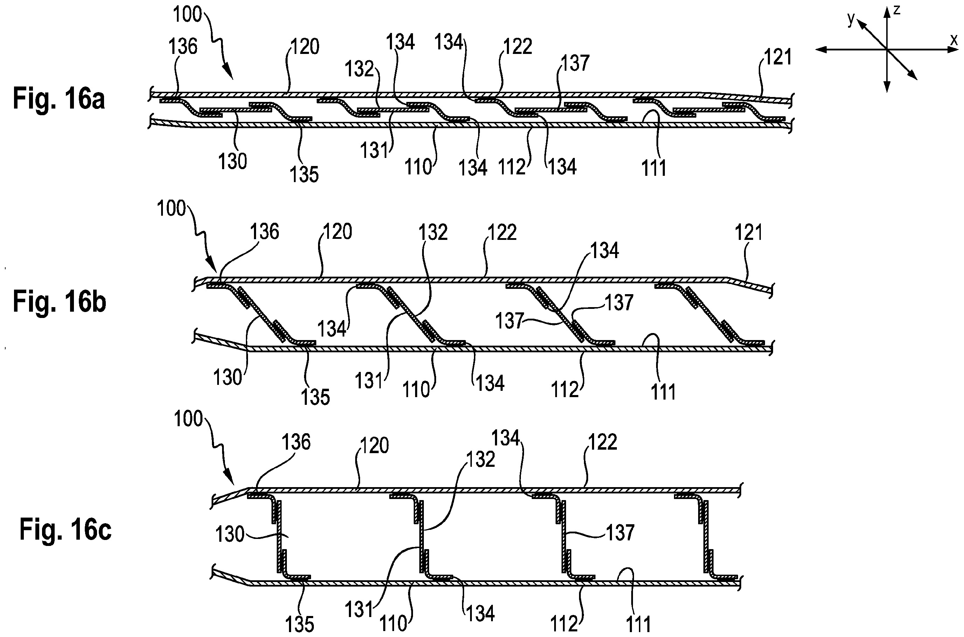

Still further, one or more areas with different properties may be achieved by providing ligaments which are assembled by attaching different pieces of material to each other so they overlap partly and partly do not overlap, such that together they form the overall ligament (instead of having one continuous material coextensive with the ligament to which additional pieces of material(s) are added only in certain areas). Examples of structures with ligaments being assembled of pieces of (different) materials are illustrated in Fig. 15A through 15C and 16A through 16C.

By having ligaments with one or more areas having properties different from the remaining ligament, the behavior of the structure with respect to e.g. bending stiffness and tensile strength can be fine-tuned to meet certain needs in different areas of the structure (e.g. the ability to accommodate readily and softly to the skin of a wearer in some areas and to be stiffer and more resistant to compression in other areas to close gaps).

If providing such areas of different properties by any of the above means, it may be especially desirable to provide them in the areas of the free intermediate portion of a ligament which are directly adjacent to the first and second ligament attachment region. The areas of the free intermediate portion which are directly adjacent to the first and second ligament attachment regions are those areas which bend upon release of the contractive force, which has provided by the stretched one or more elastic elements along the longitudinal direction of the structure, thus erecting the free intermediate portion.

The basis weight of each of the ligaments may be at least 1 g/m$^2$, or at least 2 g/m$^2$, or at least 3 g/m$^2$, or at least 5 g/m$^2$; and the basis weight may further be not more than 1000 g/m$^2$, or not more than 500 g/m$^2$, or not more than 200 g/m$^2$, or not more than 100 g/m$^2$, or not more than 50 g/m$^2$, or not more than 30 g/m$^2$.

If the tensile strength is the same throughout the ligament, the tensile strength of the ligaments may be at least 3 N/cm, or at least 5 N/cm or at least 10 N/cm. The tensile strength may be less than 100 N/cm, or less than 80 N/cm, or less than 70 N/cm, or less than 50 N/cm, or less than 40 N/cm.

If the tensile strength is the same throughout the ligament, the bending stiffness of the ligaments may be at least 0.1 mNm, or at least 0.2 mNm, or at least 0.3 mNm. The bending stiffness may be less than 500 mNm, or less than 300 mNm, or less than 200 mNm, or less than 150 mNm. Principally, for the ligaments the same considerations regarding overall softness, drape and conformability versus overall stability and robustness apply as set out above for the first and second layer. However, the bending stiffness and tensile strength of the ligaments typically has a higher impact on the overall bending resistance of the erected structure (when a force is applied along the caliper of the structure, i.e. perpendicular to the lateral and longitudinal dimension of the structure) vs. the impact of the bending stiffness and tensile strength of the first and second layer. Thus, it may be desirable that the ligaments have a higher bending stiffness and a higher tensile strength than the first and second layer.

The different ligaments in a structure may vary from each other in basis weigh and/or tensile strength, bending stiffness and the like.

**Stop aid**

It may be desirable to define a maximum shifting of the first and second layers (110, 120) relative to each upon application of the force along the longitudinal dimension of the structure (100). This can be facilitated by providing a stop aid (180; 190). However, for the present invention, the provision of such stop aid may not be required, as the maximum shifting of the first layer and the second layer relative to each other is generally defined by the contration of the one or more elastic elements. Hence, when the one or more elastic elements are completely contracted, no further shifting of first and second layer occurs. It may, however, be desirable to stop further erection by hindering further contraction of the one or more elastic elements, such that the one or more elastic elements do not fully contract. Therefore, a stop aid can be facilitated to provide a counter-force along the longitudinal dimension of the structure, such that, in the erected structure, the force applied by the one or more elastic elements in one direction along the longitudinal dimension (due to incomplete contraction of the elastic elements) is balanced against a force applied in the respective opposite direction by an appropriate stop aid.

By using a stop aid (180; 190), the structure (100) can thus be stopped in a defined erected configuration, i.e. with a defined caliper (which, however, is higher than the caliper of the structure in its flat configuration), even if the force along the longitudinal dimension is continued to be applied (due to incomplete contraction of the one or more elastic elements). The stop aid (180; 190) may facilitate that the structure (100) is stopped in the erected configuration with a certain caliper, which is higher than the caliper of the initial flat configuration but lower than the highest possible caliper which would be possible due to the longitudinal dimension of the free intermediate portion (137) of the ligaments (130).

There are many different ways to provide a stop aid (180; 190), for example:

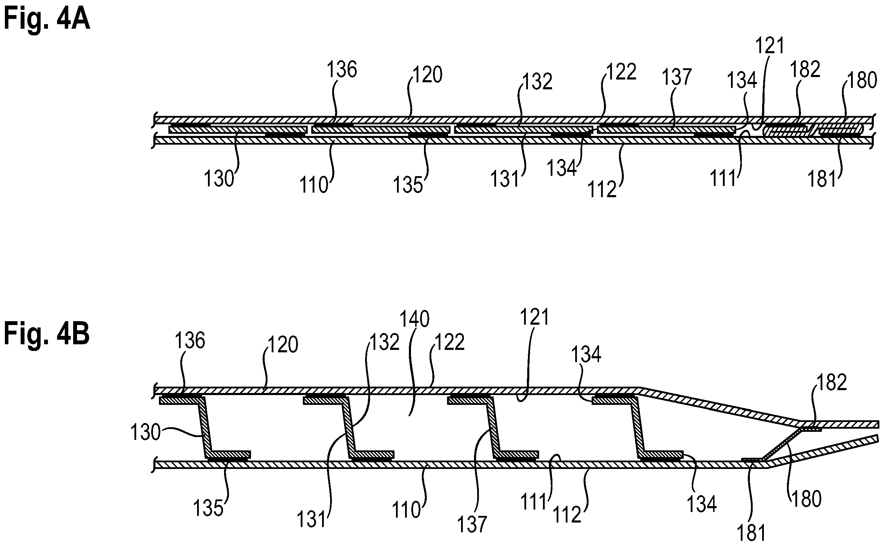

a) A layer-to-layer stop aid (180) may be provided, which extends from the first layer (110) to the second layer (120). This layer-to-layer stop aid (180) is attached to the inner surface (111) or outer surface (112) of the first layer (110) in a first layer-to-layer stop aid attachment region (181) and is further attached to the inner surface (121) or outer surface (122) of the second layer (120) in a second layer-to-layer stop aid attachment region (182). The first layer-to-layer stop aid attachment region (181) may be longitudinally spaced apart from the second layer-to-layer stop aid attachment region (182) when the structure is in its initial flat configuration. The layer-to-layer stop aid (180) is provided with a layer-to-layer stop aid leeway between the first and second layer-to-layer stop aid attachment regions (181, 182) when the structure (100) is in its initial flat configuration. The leeway may be configured in form of a slack. Upon release of the contractive force, which has provided by the stretched one or more elastic elements along the longitudinal dimension the first and second layers (110, 120) shift relative to each other along the longitudinal dimension, the structure contracts in areas where the one or more elastic elements are provided and erects, and the slack forming the layer-to-layer stop aid leeway between the first and second layer-to-layer stop aid attachment regions (181, 182) straightens out. Once the slack is flattened when the structure (100) is in its erected position, further longitudinal extension of the structure is inhibited also when the force in the longitudinal dimension is continued to be applied. An example of a layer-to-layer stop aid (180) is illustrated in Figs. 4A (initial flat configuration) and 4B (erected configuration).

Alternatively, the leeway of the layer-to-layer stop aid (180) can be generated by adapting the material between the first and second layer-to-layer stop aid attachment regions (181, 182) to create extensibility of the respective area of the layer-to-layer stop aid (180). Adapting the material can be done by modifying the material, e.g. by selfing (weakening the material of the first or second layer to render it relatively easily extensible), creating holes or using extensible materials to form the leeway. Alternatively, the layer-to-layer stop aid (180) may be made of extensible material. For such layer-to-layer stop aids (180), the material of the leeway elongates when the first and second layers (110, 120) are shifted against each other until elongation of the layer-to-layer stop aid leeway is not possible any longer (without applying an excessive amount of force, which may even rupture the structure). Hence, the material in the leeway has reached its maximum elongation i.e. it cannot be elongated further upon application of force without causing damage to the structure that limits or impedes its intended use.

The material of the leeway may also be elastic. For such structures, the layer-to-layer stop aid (180) can retract when the force is no longer applied onto the structure such that the structure can substantially "snap back" into its initial flat configuration. The difference between the provision of an elastic leeway and the one or more elastic elements comprised by or associated with the structure is that the one or more elastic elements are stretched when the structure is in its flat configuration and contracts upon erection of the structure. Contrary thereto, an elastic leeway will be relaxed in the flat configuration of the structure and stretch upon erection of the structure.

It is also possible to provide a leeway that is a combination of a slack and the provision of extensible material in the leeway, such that, upon elongation, initially the slack straightens out and subsequently, the extensible material elongates.

Compared to the attachment of the ligaments to the first and second layer and the resulting configuration, the layer-to-layer stop aid, when applied between the first and second layer and being attached to the inner surfaces of the first and second layer, does not adopt a Z-like or C-like shape having the same orientation as the ligaments with Z-like or C-like shape (otherwise, it would simply be an additional ligament). Instead, the first layer-to-layer stop aid attachment region may be between the first surface of the layer-to-layer stop aid and the inner surface of the first layer. The second layer-to-layer stop aid attachment region may be between the first surface of the layer-to-layer stop aid (i.e. on the same surface of the layer-to-layer stop aid as the first layer-to-layer stop aid attachment region) and the inner surface of the second layer. Thus, the layer-to-layer stop aid adopts a C-like shape when the structure is in its erected configuration.

Alternatively or additionally, the first layer-to-layer stop aid attachment region may be between a first surface of the layer-to-layer stop aid and the inner surface of the first layer. The second layer-to-layer stop aid attachment region may be between the second surface of the layer-to-layer stop aid (i.e. opposite surface of the layer-to-layer stop aid than the first layer-to-layer stop aid attachment region) and the inner surface of the second layer. Thus, the layer-to-layer stop aid adopts a Z-like shape configuration when the structure is in its erected configuration.

Attachment of the layer-to-layer stop aid (180) to the first and second layer (110, 120) in the first and second layer-to-layer stop aid attachment regions (181, 182) can be obtained by any means known in the art, such as adhesive, thermal bonding, mechanical bonding (e.g. pressure bonding), ultrasonic bonding, or combinations thereof.

As the first layer is attached to the topsheet (if the structure is placed on top of the topsheet), the secondary topsheet, the acquisition system and/or the absorbent core, the layer-to-layer attachment regions may actually also be between the second layer and the topsheet (if the structure is placed on top of the topsheet), the secondary topsheet, the acquisition system and/or the absorbent core, as long as all necessary requirements set out above for the layer-to-layer stop aid are fulfilled to provide a stop aid function.

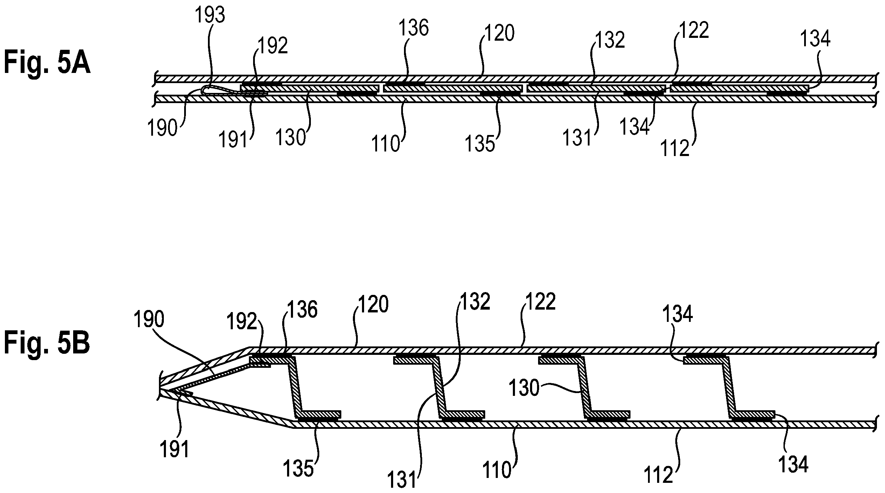

b) A layer-to-ligament stop aid (190) may be provided, which extends from the first or second layer (110, 120) to one of the ligaments (130). This layer-to-ligament stop aid (190) is attached to the inner surface (111) or outer surface (112) of the first or second layer (110, 120) in a first layer-to-ligament stop aid attachment region (191) and is further attached to the first surface (131) or second surface (132) of the ligament (130) in a second layer-to-ligament stop aid attachment region (192). The first layer-to-ligament stop aid attachment region (191) may be longitudinally spaced apart from the second layer-to-ligament stop aid attachment region (192). The layer-to-ligament stop aid (190) is provided with a layer-to-ligament stop aid leeway between the first and second layer-to-ligament stop aid attachment region (191, 192) when the structure (100) is in its initial flat configuration. The leeway may be configured in form of a slack (193). Upon release of a contractive force, which has provided by the stretched one or more elastic elements in the longitudinal dimension the first and second layer (110, 120) shift relative to each other along the longitudinal dimension, the structure contracts in areas where the one or more elastic elements are provided and erects, and the slack (193) forming the layer-to-ligament stop aid leeway between the first and second layer-to-ligament stop aid attachment regions (191, 192) straightens out. Once the slack (193) is straightened out when the structure (100) is in its erected position, further longitudinal extension of the structure is inhibited also when the force in the longitudinal dimension is continued to be applied. A layer-to-ligament stop aid (190) is shown in Figs. 5A (initial flat configuration) and 5B (erected configuration).

Alternatively or additionally, the leeway of the layer-to-ligament stop aid (190) can be generated by adapting the material between the first and second layer-to-ligament stop aid attachment regions (191, 192) to create extensibility of the respective area of the layer-to-ligament stop aid (190). Adapting the material can be done by modifying the material, e.g. by selfing (weakening the material of the first or second layer to render it relatively easily extensible), creating holes or using extensible materials to form the leeway.

Alternatively or additionally, the layer-to-ligament stop aid (190) may be made of extensible material. For such layer-to-ligament stop aids (190), the material of the leeway elongates when the first and second layers (110, 120) are shifted against each other until elongation of the layer-to-ligament stop aid leeway is not possible any longer (without applying an excessive amount of force, which may even rupture the structure). Hence, the material in the leeway has reached its maximum elongation i.e. it cannot be elongated further upon application of force without causing damage to the structure that limits or impedes its intended use.

The material of the leeway may also be elastic. For such structures, the layer-to-ligament stop aid (190) can retract when the force is no longer applied onto the structure such that the structure can substantially "snap back" into its initial flat configuration.

For the material properties and appropriate selection of extensible or elastic leeways, and the difference between elastic leeways and the one or more elastic elements comprised by or associated with the structure, the same considerations apply as are set out above for the layer-on-layer stop aid.

It is also possible to provide a leeway that is a combination of a slack and the provision of extensible material in the leeway, such that, upon elongation, initially the slack straightens out and subsequently, the extensible material elongates.

Attachment of the layer-to-ligament stop aid (190) to the first and second layer (110, 120) in the first and second layer-to-layer stop aid attachment regions (181, 182) can be obtained by any means known in the art, such as adhesive, thermal bonding, mechanical bonding (e.g. pressure bonding), ultrasonic bonding, or combinations thereof. Generally, the layer-to-ligament stop aid (190) may be attached in the first and second layer-to-ligament stop aid attachment regions such that the layer-to-ligament stop aid extends along or adjacent to one of the longitudinal edges of the at least one ligament (130) or, alternatively, such that it extends between the longitudinal edges of the at least one ligament.

As the first layer is attached to the topsheet (if the structure is placed on top of the topsheet), the secondary topsheet, the acquisition system and/or the absorbent core, the layer-to-ligament attachment regions may actually also be between the second layer and the topsheet (if the structure is placed on top of the topsheet), the secondary topsheet, the acquisition system and/or the absorbent core, as long as all necessary requirements set out above for the layer-to-ligament stop aid are fulfilled to provide a stop aid function.

c) The structure (100) may comprise an enveloping stop aid which encircles at least a portion of the first and second layer (110, 120) and the ligaments (130) provided between the first and second layer in the respective portion. This enveloping stop aid is attached to the first layer (110), in one or more enveloping stop aid attachment region. Attaching the enveloping stop aid to only one of the first layer (110).

The enveloping stop aid is attached to itself to form a closed loop with a defined circumference around at least a portion of the first and second layer with the ligaments in between. Alternatively or in addition, if the structure is placed on top of the topsheet and the structure is enwrapped by a wrap-sheet as described above, the wrap-sheet may serve as an enveloping stop aid. Also, the wrap-sheet, if not fully enwrapping the structure, may, in conjunction with the topsheet, to which it is attached, serve as an enveloping stop aid. The enveloping stop aid may (probably together with portions of the topsheet) encircle the first and second layer (110, 120) along the lateral dimension (and the caliper).

The circumference of the enveloping stop aid defines the maximum shifting of the first layer (110) and the second layer (120) relative to each other upon release of a contractive force, which has provided by the stretched one or more elastic elements along the longitudinal dimension.

When the structure (100) is in its initial flat configuration, the enveloping stop aid is loose around the first and second layer (and the respective ligaments between the first and second layer). Upon release of a contractive force, which has provided by the stretched one or more elastic elements along the longitudinal dimension of the structure, the structure erects until the enveloping stop aid fits tightly around the first and second layer (and the respective ligaments between the first and second layer), which will stop further shifting of the first layer relative to the second layer also if the force along the longitudinal dimension is continued to be applied. To assist in avoiding overexpansion of the structure (100), the circumference of the enveloping stop aid may be such that further shifting of the first layer (110) relative to the second layer (120) along the longitudinal dimensions is inhibited before the ligaments (130) are in their fullest upright position.

*General considerations for the layer-to-layer stop aid, the layer-to-ligament stop aid and, if expressly mentioned, the enveloping stop aid:*

The layer-to-layer stop aid and/or the layer-to-ligament stop aid may be non-elastic or highly non-elastic (apart from the leeway, if the leeway is provided by modifying the material to render it elastically extensible). Also the layer-to-layer stop aid and/or the layer-to-ligament stop aid may be non-extensible or highly non-extensible (apart from the leeway, if the leeway is provided by modifying the material to render it extensible).

The layer-to-layer stop aid and/or the layer-to-ligament stop aid and/or enveloping stop aid can be made of a sheet-like material, such as nonwoven, film, paper, sheet-like foam, woven fabric, knitted fabric, or combinations of these materials. Combinations of these materials may be laminates, e.g. a laminate of a film and a nonwoven. The layer-to-layer stop aid and/or the layer-to-ligament stop aid and/or enveloping stop aid may also be made of a cord- or string-like material.

The layer-to-layer stop aid and/or the layer-to-ligament stop aid and/or enveloping stop aid is not necessarily intended to contribute to the resistance of the structure against a force exerted onto the structure in the thickness-direction. However, the basis weight, tensile strength and bending stiffness of the layer-to-layer stop aid and/or the layer-to-ligament stop aid and/or enveloping stop aid should be sufficiently high to avoid inadvertent tearing of the layer-to-layer stop aid and/or the layer-to-ligament stop aid and/or enveloping stop aid upon expansion of the structure.

If the layer-to-layer stop aid and/or the layer-to-ligament stop aid and/or enveloping stop aid is made of a sheet-like material, the basis weight of the layer-to-layer stop aid and/or the layer-to-ligament stop aid and/or enveloping stop aid may be at least 1 g/m$^2$, or at least 2 g/m$^2$, or at least 3 g/m$^2$, or at least 5 g/m$^2$; and the basis weight may further be not more than 500 g/m$^2$, or not more than 200 g/m$^2$, or not more than 100 g/m$^2$, or not more than 50 g/m$^2$, or not more than 30 g/m$^2$.

If the layer-to-layer stop aid and/or the layer-to-ligament stop aid and/or enveloping stop aid is made of a cord- or string-like material, the basis weight of the layer-to-layer stop aid and/or the layer-to-ligament stop aid and/or enveloping stop aid may be at least 1 gram per meter (g/m), or at least 2 g/m, or at least 3 g/m, or at least 5 g/m; and the basis weight may further be not more than 500 g/m, or not more than 200 g/m, or not more than 100 g/m, or not more than 50 g/m, or not more than 30 g/m.

The basis weight of the layer-to-layer stop aid and/or the layer-to-ligament stop aid may be less than the basis weight of the ligaments, for example the basis weight of the layer-to-layer stop aid and/or the layer-to-ligament stop aid may be less than 80%, or less than 50% of the basis of the ligaments (if the ligaments vary in basis weight, then these values are with respect to the ligament(s) with the lowest basis weight).

The tensile strength of the layer-to-layer stop aid may be at least 2 N/cm, or at least 4 N/cm or at least 5 N/cm. The tensile strength may be less than 100 N/cm, or less than 80 N/cm, or less than 50 N/cm, or less than 30 N/cm, or less than 20 N/cm.

The bending stiffness of the layer-to-layer stop aid and/or the layer-to-ligament stop aid and/or enveloping stop aid may be at least 0.1 mNm, or at least 0.2 mNm, or at least 0.3 mNm. The bending stiffness may be less than 200 mNm, or less than 150 mNm, or less than 100 mNm, or less than 50 mNm, or less than 10 mNm, or less than 5 mNm. These values apply to sheet-like layer-to-layer stop aids and/or layer-to-ligament stop aids and/or enveloping stop aids, for cord- or string-like layer-to-layer stop aids and/or layer-to-ligament stop aids and/or enveloping stop aids, the bending stiffness is generally not seen as critical.

The tensile strength of the layer-to-layer stop aid and/or the layer-to-ligament stop aid and/or enveloping stop aid may be lower than the tensile strength of the ligaments, for example the tensile strength of the layer-to-layer stop aid may be less than 80%, or less than 50% of the tensile strength of the ligaments (if the ligaments vary in tensile strength, then these values are with respect to the ligament(s) with the lowest tensile strength).

The bending stiffness of the layer-to-layer stop aid and/or the layer-to-ligament stop aid and/or enveloping stop aid (when made of sheet-like material) may be lower than the bending stiffness of the ligaments, for example the bending stiffness of the layer-to-layer stop aid and/or the layer-to-ligament stop aid and/or enveloping stop aid may be less than 80%, or less than 50% of the bending stiffness of the ligaments (if the ligaments vary in bending stiffness, then these values are with respect to the ligament(s) with the lowest bending stiffness).

**Test methods:**

**Tensile Strength**

Tensile Strength is measured on a constant rate with extension tensile tester Zwick Roell Z2.5 with computer interface, using TestExpert 11.0 Software, as available from Zwick Roell GmbH &Co. KG, Ulm, Germany. A load cell is used for which the forces measured are within 10% to 90% of the limit of the cell. Both the movable (upper) and stationary (lower) pneumatic jaws are fitted with rubber faced grips, wider than the width of the test specimen. All testing is performed in a conditioned room maintained at about 23°C + 2°C and about 45 % $\pm$ 5% relative humidity.

With a die or razor knife, cut a material specimen which is 25.4 mm wide and 100 mm long. For the present invention, the length of the specimen correlates to the longitudinal dimension of the material within the structure.

If the ligament is smaller than the size of the material specimen specified in the previous paragraph, the material specimen may be cut from a larger piece such as the raw material used for making the ligaments. Care should be taken to correlate the orientation of such specimen accordingly, i.e. with the length o the specimen correlating to the longitudinal dimension of the material within the structure. However, if the ligament has a width somewhat smaller than 25.4 mm (e.g. 20 mm, or 15 mm) the width of the specimen can be accordingly smaller without significantly impacting the measured tensile strength.

If the ligament comprises different materials in different regions, the tensile strength of each material can be determined separately by taking the respective raw materials. It is also possible to measure the tensile strength of the overall ligament. However, in this case, the measured tensile test will be determined by the material within the ligament which has the lowest tensile strength.

Precondition the specimens at about 23 °C $\pm$ 2°C and about 45 % $\pm$ 5% relative humidity for 2 hours prior to testing.

For analyses, set the gauge length to 50 mm. Zero the crosshead and load cell. Insert the specimen into the upper grips, aligning it vertically within the upper and lower jaws and close the upper grips. Insert the specimen into the lower grips and close. The specimen should be under enough tension to eliminate any slack, but less than 0.025 N of force on the load cell.

Program the tensile tester to perform an extension test, collecting force and extension data at an acquisition rate of 50 Hz as the crosshead raises at a rate of 100 mm/min until the specimen breaks. Start the tensile tester and data collection. Program the software to record

Peak Force (N) from the constructed force (N) verses extension (mm) curve. Calculate tensile strength as:

$$\text{Tensile Strength} = \text{Peak Force (N) / width of specimen (cm)}$$

$$\text{For rope/string like materials: tensile strength} = \text{peak force (N)}$$

Analyze all tensile Specimens. Record Tensile Strength to the nearest 1 N/cm. A total of five test samples are analyzed in like fashion. Calculate and report the average and standard deviation of Tensile Strength to the nearest 1 N/cm for all 5 measured specimens.

**Bending Stiffness**

Bending stiffness is measured using a Lorentzen & Wettre Bending Resistance Tester (BRT) Model SE016 instrument commercially available from Lorentzen & Wettre GmbH, Darmstadt, Germany. Stiffness off the materials (e.g. ligaments and first and second layer) is measured in accordance with SCAN-P 29:69 and corresponding to the requirements according to DIN 53121 (3.1 "Two-point Method"). For analysis a 25.4 mm by 50 mm rectangular specimen was used instead of the 38.1 mm by 50 mm specimen recited in the standard. Therefore, the bending force was specified in mN and the bending resistance was measured according to the formula present below.

The bending stiffness is calculated as follows:

$$S_b = \frac{60 \times F \times l^2}{\pi \times \alpha \times b}$$

with:

$S_b$ = bending stiffness in mNm
F = bending force in N
1 = bending length in mm
$\alpha$ = bending angle in degrees
b = sample width in mm

With a die or razor knife, cut a specimen of 25.4 mm by 50 mm whereby the longer portion of the specimen corresponds to the lateral dimension of the material when incorporated into a structure. If the materials are relatively soft, the bending length "1" should be 1 mm. However, if the materials are stiffer such that the load cell capacity is not sufficient any longer for the measurement and indicates "Error", the bending length "1" has to be set at 10 mm. If with a bending length "1" of 10 mm, the load cell again indicates "Error", the bending length "1" may be chosen to be more than 10 mm, such as 20 mm or 30 mm. Alternatively (or in addition, if needed), the bending angle may be reduced from 30° to 10°.

For the ligament data given in Table 1, the bending length "1" was 10 mm. For the 1st and 2nd layer materials the bending length "1" was 1 mm. The bending angel has been 30° for the ligaments as well as for the 1 and 2nd layer.

Precondition the specimen at about 23 °C $\pm$ 2°C and about 45 % $\pm$ 5% relative humidity for two hours prior to testing.

Regarding the size of the ligament and the procedure in case the size of the ligament is smaller than the size of the specimen, the same applies as set out above for the tensile test.

**Table 1:** Basis weight, tensile strength and bending stiffness of suitable first and second layers materials:

| Example No. | 1 | 2 | 3 | 4 |
|---|---|---|---|---|
| Basis weight of 1st and 2nd layer | 13 g/m$^2$ | 15 g/m$^2$ | 17 g/m$^2$ | 25 g/m$^2$ |
| Bending stiffness of 1st and 2nd layer | 0.3 mNm | 0.4 mNm | 0.5 mNm | 0.9 mNm |
| Tensile strength of 1st and 2nd layer | 6.9 N/cm | 7.9 N/cm | 7.8 N/cm | 8.1 N/cm |

Example materials 1 to 4 are all spunbond polypropylene nonwovens.

**Table 2:** Basis weight, tensile strength and bending stiffness of suitable ligament materials:

| Example No. | 1 | 2 | 3 | 4 |
|---|---|---|---|---|
| Basis weight of ligaments | 40 g/m$^2$ | 43 g/m$^2$ | 51 g/m$^2$ | 60 g/m$^2$ |
| Bending stiffness of ligaments | 10.8 mNm | 36.3 mNm | 52.6 mNm | 105.3 mNm |
| Tensile strength of ligaments | 16.2 N/cm | 16.1 N/cm | 18.8 N/cm | 26.1 N/cm |

Except for Example 1 (40 g/m$^2$ material), all ligament materials were spunbond PET nonwovens. Example 1 was a spunbond polypropylene material.

**Method to measure ligament caliper**

Average Measured caliper is measured using a Mitutoyo Absolute caliper device model ID-C1506, Mitutoyo Corp., Japan.

A sample of the material used for the ligaments with a sample size of 40 mm x 40 mm is cut. If the samples are taken from a ready-made structure and the size of the ligaments is smaller than 40 mm x 40 mm, the sample may be assembled by placing two or more ligaments next to each other with no gap and no overlap between them. Precondition the specimens at about 23 °C $\pm$ 2°C and about 45 % $\pm$ 5% relative humidity for 2 hours prior to testing.

Place the measuring plate on the base blade of the apparatus. Zero the scale when the probe touches the measuring plate (Measuring plate 40 mm diameters, 1.5 mm height and weight of 2.149 g). Place the test piece on the base plate. Place the measurement plate centrally on top of the sample without applying pressure. After 10 sec. move the measuring bar downwards until the probe touches the surface of the measuring plate and read the caliper from the scale to the nearest 0.01 mm.

**Method of measuring modulus of the structure**

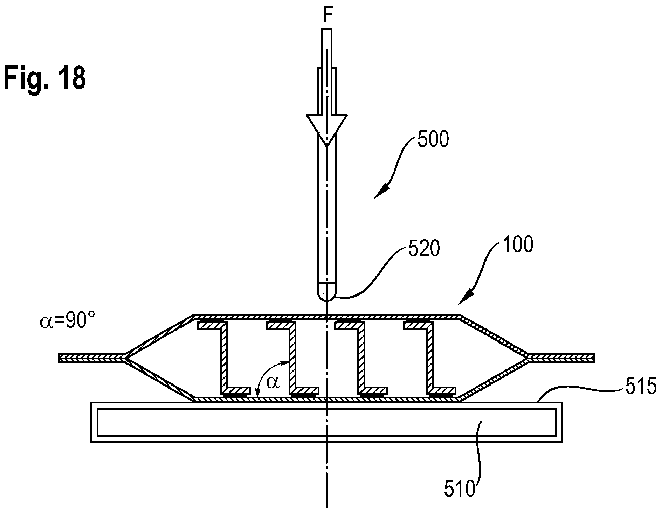

The modulus of the structure is measured on a constant rate of structure compression using a tensile tester with computer interface (a suitable instrument is the Zwick Roell Z2.5 using TestExpert 11.0 Software, as available from Zwick Roell GmbH &Co. KG, Ulm, Germany) using a load cell for which the forces measured are within 10% to 90% of the limit of the cell. The movable upper stationary pneumatic jaw is fitted with rubber faced grip to securely clamp the plunger plate (500). The stationary lower jaw is a base plate (510) with dimensions of 100mm x 100mm. The surface of the base plate (510) is perpendicular to the plunger plate (500). To fix the plunger plate (500) to the upper jaw, lower the upper jaw down to 20mm above the upper surface (515) of the base plate (510). Close the upper jaw and make sure the plunger plate (500) is securely tightened. Plunger plate (500) has a width of 3.2mm and a length of 100mm. The edge (520) of the plunger plate (500) which will contact the structure has a curved surface with an impacting edge radius of r = 1.6mm. For analysis, set the gauge length to at least 10% higher than the caliper of the structure in its erected configuration (see Fig 18). Zero the crosshead and load cell. The width of the plunger plate (500) should be parallel with the transverse direction of the structure.

Precondition samples at about 23 °C $\pm$ 2°C and about 45% RH $\pm$ 5% RH relative humidity for 2 hours prior to testing. The structure is placed on the base plate, is transformed into its erected configuration and fixed in its erected configuration with substantially maximum possible caliper to the base plate with the outer surface of its first (lower) layer facing towards the upper surface (515) of the base plate (510). The structure can be fixed to the upper surface of the base plate, e.g. by placing adhesive tapes on the lateral edges of the structure's first (lower) layer and fix the tapes to the upper surface of the base plate.

Program the tensile tester to perform a compression test, collecting force and travel distance data at an acquisition rate of 50 Hz as the crosshead descends at a rate of 50 mm/min from starting position to 2mm above base plate (safety margin to avoid destruction of load cell).

If the modulus of the structure is measured directly in an area where a ligament is placed, the force P [N] is the force when the indentation depth h [mm] of the plunger plate into the structure is equal to 50% of the longitudinal dimension of the free intermediate portion of the ligament below the plunger plate.

If the modulus of the structure is measured between two neighboring ligaments, the force P [N] is the force when the indentation depth h [mm] of the plunger plate into the structure is equal to 50% of the longitudinal dimension of the free intermediate portion of the two ligaments nearest to the plunger plate (i.e. the ligaments on each side of the plunger plate as seen along the longitudinal structure dimension). If the free intermediate portion of the two neighboring ligaments, between which the modulus is measured, differ from each other with respect to the longitudinal dimension of their free intermediate portions, the average value over these two free intermediate portions is calculated and the indentation depth h [mm] of the plunger plate into the structure is equal to 50% of this average free longitudinal dimension.

A total of three test specimens are analyzed in like fashion.

The modulus E [N/mm$^2$] is calculated as follows:

$$E = \frac{3P}{8rh}.$$

With r being the impacting edge radius of the plunger plate, i.e. r = 1.6mm

Calculate and report the average of modulus E for all 3 measured specimens.

All testing is performed in a conditioned room maintained at about 23°C + 2°C and about 45%RH $\pm$ 5% relative humidity.

**Example structures**

*Note: the following example structures will often be too small to be suitable for use in absorbent articles of the present invention. Moreover, no elastic elements are provided in the structure. The examples are mainly intended to*

*exemplify and explain in more detail how a "cell forming structure" can be made in general. Changing and adapting the dimensions, material and configuration of the structures described below in order to render them suitable for use in absorbent articles of the present invention is a matter of simple routine work based on the detailed description provided above.*

Making of example structures:

For each structure, cut 2 pieces of nonwovens with longitudinal dimension of 150 mm and a lateral dimension of 25 mm, with a die or razor knife. These nowovens will become the first and second layers of the structure. Cut 4 ligaments (Examples 1, 2, 4 and 5), respectively 6 ligaments (Example 3) with a longitudinal dimension of 10 mm (which will result in a longitudinal dimension of the free intermediate portion of 4 mm in the final structures, while 3 mm on each lateral edge are attached to the first and second layer, respectively) and a lateral dimension of 25 mm, with a die or razor knife. Apply a double sided tape (e.g. 3M Double sided medical tape 1524-3M ($44g/m^2$) available from 3M) with length of 3 mm and width of 25 mm on the first surface of the each ligament adjacent the side edge, which will become the first lateral edge of the ligament in the final structure and a second tape on the second surface of each ligament adjacent the side edge, which will become the second lateral edge of the ligament in the final structure. The width of the tape is aligned with the lateral dimension of the ligaments.

Remove one release tape from the ligaments and attach the tape to the first layer the first layer such that the lateral dimension of the ligament is aligned with the lateral dimension of the first layer.

For Example 1: Place the first, second, third and fourth ligaments such that the spacing between neighboring ligaments is 10 mm when the ligaments are lying flat on the first layer.

For Example 2, 4 and 5: Place the first, second, third and fourth ligaments such that the spacing between neighboring ligaments is 5mm when the ligaments are lying flat on the first layer.

For Example 3: In this example, 6 ligaments where positioned between the first and second layer. Place the first to sixth ligaments such that there is no spacing between neighboring ligaments and neighboring ligaments overlap by 3 mm with each other when the ligaments are lying flat on the first layer.

For all examples, the ligaments should be positioned accordingly, to leave sufficient space at the lateral edges of the first and second layer to allow attaching the first layer to the second layer in the manner described below.

For all Examples structures, the 1st and 2nd layer were made of the material of Example 2 of Table 1. The ligaments for the Example structures 1, 2, 3 and 5 were made of the ligament material of Example 4 of Table 2, while the ligaments of Example structure 4 were made of the ligament material of Example 1 of Table 2.

Remove the remaining release layers from the remaining tape pieces on all four ligaments and attach the second layer on top of the first layer and the ligaments such that the second layer is congruent with the first layer.

To bond the first layer to the second layer in the areas longitudinally outwardly from the area where the ligaments are placed (stop aid), two double-sided tapes (e.g. 3M Double sided medical tape 1524-3M ($44g/m^2$) available from 3M) having a length of 3 mm and a width of 25 mm are provided. A first tape is attached to the first layer towards one of the first layer's lateral edges such that the distance between this first tape and the adjacent ligament is 20 mm. The second tape is attached to the first layer towards the respective other lateral edge of the first layer such that the distance between this second tape and the respective adjacent ligament is 20 mm. The width of the first and second tape is aligned with the lateral dimension of the first layer. Pay attention that the first and second tapes are not attached to the second layer before the structure has been transformed into its erected configuration (see next step).

Stretch the resulting cell forming structure along the longitudinal dimension into the erected configuration such that the first and second layer shift relative to each other and the ligaments move in upright position of 90° relative to the first and second layer. Notably, the 90° does not apply to the area longitudinally outwardly from the outermost ligaments (viewed along the longitudinal dimension) because the first and second layers follow a tapered path in this area until the point where they coincide with each other (see e.g. Fig. 1B). Maintain the structure in its erected configuration and attach the first layer to the second layer via the first and second tape to fix the structure in its erected configuration. Release the force and allow the structure to relax.

For the Structure Modulus Test, place the assembled cell forming structure flat and unstretched under the plunger plate. Stretch the cell forming structure such that in the erected configuration the ligaments are approximately at an angle of 90° versus the first and second layer in the areas between neighboring ligaments.

The plunger plate was placed centrally in the space between the two center ligaments of the structure, except for Example 5, where the plunger plate was placed centrally directly in the location where one of the two centrally positioned (viewed along the longitudinal direction of the structure) ligaments is. The erected structure was fixed to the base plate using tape. The test procedure set out above for the structure modulus was followed.

**Table 3:** Modulus of the structure

| Example Structure No. | 1 | 2 | 3 | 4 | 5 |
|---|---|---|---|---|---|
| Modulus [N/mm$^2$] | 0.028 | 0.039 | 0.107 | 0.005 | 0.151 |

The data show that the measured modulus of a structure will be different depending e.g. on whether the modulus is measured between neighboring ligaments (Examples 1 to 4), or if it is measured directly in the location where a ligament is attached to the first and second layer. For a given structure, between neighboring ligaments, the modulus will typically be lower than directly at a ligament.

**Claims**

1. An absorbent article (20) having a longitudinal dimension and a transverse dimension and comprising
   a liquid pervious topsheet (24),
   a liquid impervious backsheet (26); and
   an absorbent core (28) disposed between the topsheet (24) and the backsheet (26); the absorbent article (20) further comprising a structure (100) positioned above the absorbent core (28) and underneath the topsheet (24) wherein the structure (100) is attached to the component of the absorbent article (20) underneath the structure (100), the structure (100) having a longitudinal dimension and a lateral dimension and a caliper, and comprising one or more elastic elements (195, 196); and/or the structure (100) is associated with one or more elastic elements (195, 196); wherein the structure (100) comprises a first and a second layer (110, 120), each layer having an inner (111, 121) and an outer surface (112, 122), a longitudinal dimension parallel to the longitudinal dimension of the structure (100) confined by first and second spaced apart lateral edges, and a lateral dimension parallel to the lateral dimension of the structure (100) confined by two spaced apart longitudinal edges, wherein the first and second layer (110, 120) at least partly overlap each other, wherein the second layer (120) is able to shift relative to the first layer (110) along the longitudinal structure dimension upon release of a contractive force, which has provided by the stretched one or more elastic elements along the longitudinal dimension,
   the structure (100) further comprising ligaments (130) provided between the first and second layer (110, 120) in at least a part of the region where the first and second layer overlap each other,
   each ligament (130) has a longitudinal dimension confined by two spaced apart lateral ligament edges (134), and a lateral dimension confined by two spaced apart longitudinal ligament edges,
   each ligament (130) being attached to the inner surface (111) of the first layer (110) with a portion at or adjacent to one of the ligament's lateral edges (134) in a first ligament attachment region (135) and being attached to the inner surface (121) of the second layer (120) with a portion at or adjacent to the ligament's other lateral edge (134) in a second ligament attachment region (136), the region of each ligament between the first and second ligament attachment region (135, 136) forming a free intermediate portion (137),
   the ligaments (130) being spaced apart from one another along the longitudinal dimension of the structure (100), and the attachment of all ligaments (130) to the first and second layer (110, 120) in the first and second ligament attachment regions (135, 136) is such that the free intermediate portions (137) of the ligaments (130) are able to convert from an initial flat configuration to an erected configuration upon release of a contractive force, which has provided by the stretched one or more elastic elements along the longitudinal dimension of the structure (100), thus converting the structure as a whole from an initial flat configuration into an erected configuration, wherein the erection is in the direction perpendicular to the longitudinal and the lateral dimension of the structure, thus increasing in caliper, the structure (100) being in the flat configuration when the absorbent article (20) is flattened out, wherein the one or more elastic elements (195, 196) are stretched along the longitudinal dimension of the structure (100),
   whereby contraction of the one or more elastic elements (195, 106) applies a force along the longitudinal dimension of the structure (100), thus converting the structure into the erected, three-dimensional configuration, wherein the structure has a higher caliper in its erected configuration compared to the caliper of the structure in its flat configuration, the erected structure spacing the topsheet (24) away from the absorbent core (28).

2. The absorbent article of claim 1, wherein the liquid pervious topsheet (24) is extensible in the longitudinal and/or transverse dimension of the absorbent article (20), such that the liquid pervious topsheet (24) does not obstruct conversion of the structure (100) from its flat configuration into its erected configuration.

3. The absorbent article of any of the preceding claims, wherein a secondary topsheet is positioned underneath the structure and above the absorbent core.

4. An absorbent article (20) having a longitudinal dimension and a transverse dimension and comprising a liquid pervious topsheet (24), having a body-facing surface and a garment-facing surface, a liquid impervious backsheet (26); and

an absorbent core (28) disposed between the topsheet (24) and the backsheet (26); the absorbent article (20) further comprising a structure (100) positioned above the topsheet (24) and attached to the wearer-facing surface of the topsheet, the structure having a longitudinal dimension and a lateral dimension and a caliper, and comprising one or more elastic elements (195, 196); and/or the structure is associated with one or more elastic elements;

wherein the structure (100) comprises a first and a second layer (110, 120), each layer having an inner (111, 121) and an outer surface (112, 122), a longitudinal dimension parallel to the longitudinal dimension of the structure (100) confined by first and second spaced apart lateral edges, and a lateral dimension parallel to the lateral dimension of the structure (100) confined by two spaced apart longitudinal edges, wherein the first and second layer (110, 120) at least partly overlap each other, wherein the second layer (120) is able to shift relative to the first layer (110) along the longitudinal structure dimension upon release of a contractive force, which has provided by the stretched one or more elastic elements along the longitudinal dimension,

the structure (100) further comprising ligaments (130) provided between the first and second layer (110, 120) in at least a part of the region where the first and second layer overlap each other,

each ligament (130) has a longitudinal dimension confined by two spaced apart lateral ligament edges (134), and a lateral dimension confined by two spaced apart longitudinal ligament edges,

each ligament (130) being attached to the inner surface (111) of the first layer (110) with a portion at or adjacent to one of the ligament's lateral edges (134) in a first ligament attachment region (135) and being attached to the inner surface (121) of the second layer (120) with a portion at or adjacent to the ligament's other lateral edge (134) in a second ligament attachment region (136), the region of each ligament between the first and second ligament attachment region (135, 136) forming a free intermediate portion (137),

the ligaments (130) being spaced apart from one another along the longitudinal dimension of the structure (100), and the attachment of all ligaments (130) to the first and second layer (110, 120) in the first and second ligament attachment regions (135, 136) is such that the free intermediate portions (137) of the ligaments (130) are able to convert from an initial flat configuration to an erected configuration upon release of a contractive force, which has provided by the stretched one or more elastic elements along the longitudinal dimension of the structure (100), thus converting the structure as a whole from an initial flat configuration into an erected configuration, wherein the erection is in the direction perpendicular to the longitudinal and the lateral dimension of the structure, thus increasing in caliper, the structure (100) being in the flat configuration when the absorbent article (20) is flattened out, wherein the one or more elastic elements (195, 196) are stretched along the longitudinal dimension of the structure,

whereby contraction of the one or more elastic elements (195, 106) applies a force along the longitudinal dimension of the structure (100), thus converting the structure into the erected, three-dimensional configuration, wherein the structure has a higher caliper in its erected configuration compared to the caliper of the structure in its flat configuration.

5. The absorbent article of any of the preceding claims, wherein the outer surface (112) of the first layer (110) is attached to any of the following: the topsheet, the secondary topsheet underneath the structure, the acquisition system underneath the structure, and/or the absorbent core underneath the structure.

6. The absorbent article of any of the claims 1 to 4, wherein the first layer (110) of the structure is formed by any of the following: a portion of the topsheet, a portion of the secondary topsheet underneath the structure, a portion of the acquisition system underneath the structure, or a portion of the absorbent core underneath the structure.

7. The absorbent article of any of the preceding claims, wherein the one or more elastic elements (195, 196) form one or more portions of the second layer (120).

8. The absorbent article of any of the preceding claims, wherein the one or more elastic elements (195, 196) are attached to the second layer (120).

9. The absorbent article of any of the preceding claims, wherein the second layer (120) is permanently attached (197) to the first layer (110), to the topsheet, to the secondary topsheet, to the acquisition system and/or to the absorbent core longitudinally outboard of the second ligament attachment region (136) which is closest to the first lateral edge of the structure and wherein the one or more elastic elements (196) at least form a portion of the second layer (120) between the second ligament attachment region (136) which is closest to the first lateral edge of the structure and the attachment (197) of the second layer (120) to the first layer (110), to the topsheet, to the secondary topsheet, to the acquisition system and/or to the absorbent core.

10. The absorbent article of any of the preceding claims, wherein the second layer (120) is permanently attached (197) to the first layer (110), to the topsheet, to the secondary topsheet, to the acquisition system and/or to the absorbent core longitudinally outboard of the second ligament attachment region (136) which is closest to the first lateral edge of the structure (100) and wherein the one or more elastic element (196) is attached to the second layer (120) at least between the second ligament attachment region (136) which is closest to the first lateral edge of the structure and the attachment of the second layer (120) to the first layer (110), to the topsheet, to the secondary topsheet, to the acquisition system and/or to the absorbent core.

11. The absorbent article of any of the preceding claims, wherein, in the flat configuration of the structure (100), the second layer (120) is releasably attached (198) to the first layer (110), to the topsheet, to the secondary topsheet, to the acquisition system and/or to the absorbent core in a location which is on the side of the one or more elastic elements (195) along the longitudinal dimension of the structure which is opposite from the side where the second layer (120) is permanently attached (197) to the first layer, to the topsheet, to the secondary topsheet, to the acquisition system and/or to the absorbent core, and wherein release of the releasable attachment (198) enables the one or more elastic elements (195) to contract and thereby convert the structure from its flat configuration into its erected configuration.

12. The absorbent article of any of the preceding claims, wherein the one or more elastic elements (195) form (a) portion(s) of the second layer (120) between adjacent second ligament attachment regions (136).

13. The absorbent article of any of the preceding claims, wherein the one or more elastic elements are attached to the second layer (120) between adjacent second ligament attachment regions (136).

14. The absorbent article of claim 12 or 13, wherein the second layer (120) is releasably attached (198) to the first layer (110), to the topsheet, to the secondary topsheet, to the acquisition system and/or to the absorbent core on both sides of the one or more elastic elements (195) along the longitudinal dimension of the structure and wherein release of the releasable attachments (198) enables the one or more elastic elements (195) to contract and thereby convert the structure from its flat configuration into its erected configuration.

15. The absorbent article of any of the preceding claims, wherein, in the structure, the ligaments (130), in their erected configuration, either adopt a Z-like shape, a C-like shape, a T-like shape, or a Double-T-like shape.

16. The absorbent article of any of the preceding claims, wherein the structure comprises one or more stop aid(s) (180, 190) which define(s) the maximum shifting of the second layer (120) relative to the first layer (110) along the longitudinal dimension when the force along the longitudinal dimension is applied due to contraction of the one or more elastic elements (195, 196), wherein the maximum shifting defined by the stop aid (180, 190) is less than the maximum shifting provided by the ligaments (130) in the absence of such stop aid.

17. The absorbent article of claim 16, wherein the one or more stop (180, 190) aid is selected from the group consisting of:

a. a layer-to-layer stop aid (180) extending from the first layer to the second layer and being attached to the first layer in a first layer-to-layer stop aid attachment region and being further attached to the second layer in a second layer-to-layer stop aid attachment region, wherein the layer-to-layer stop aid is provided with a layer-to-layer stop aid leeway between the first layer-to-layer stop aid attachment region and the second layer-to-layer stop aid attachment region when the structure is in its initial flat configuration, said leeway being able to straighten out and/or extend when the structure is transferred into its erected configuration; and

b. a layer-to-ligament stop aid (190) extending from the first or second layer to one of the ligaments and being attached to the first or second layer in a first layer-to-ligament stop aid attachment region and being attached to the ligament in a second layer-to-ligament stop aid attachment region, wherein the layer-to-ligament stop aid is provided with a layer-to-ligament stop aid leeway between the first layer-to-ligament stop aid attachment region and the second layer-to-ligament stop aid attachment region when the structure is in its initial flat configuration, said leeway being able to straighten out and/or extend when the structure is transferred into its erected configuration; and

c. an enveloping stop aid encircling a least a portion of the first and second layer and of the ligaments between the first and second layer, wherein the enveloping stop aid is attached to the first layer, the second layer and/or one or more ligaments in at least one enveloping stop aid attachment region and wherein the enveloping stop aid is further attached to itself to form a closed loop with a defined circumference around a least a portion of the first and second layer and of the ligaments between the first and second layer, wherein the circumference of the enveloping stop aid defines the maximum caliper of the structure in its erected configuration; wherein the caliper is perpendicular to the lateral and longitudinal dimension of the structure; and

d. any combinations of a) to c).

18. The absorbent article of any of the preceding claims, wherein, in the structure, the areas where the ligaments are located and the areas between neighboring ligaments have a modulus of from about 0.01 N/mm$^2$ to about 0.3 N/mm$^2$ when measured according to the test method set out herein.

**Patentansprüche**

1. Absorptionsartikel (20) mit einer Längsabmessung und einer Querabmessung und umfassend

eine flüssigkeitsdurchlässige Oberschicht (24),

eine flüssigkeitsundurchlässige Unterschicht (26); und

einen Absorptionskern (28), der zwischen der Oberschicht (24) und der Unterschicht (26) angeordnet ist; wobei der Absorptionsartikel (20) ferner eine Struktur (100) umfasst, die über dem Absorptionskern (28) und unterhalb der Oberschicht (24) angeordnet ist, wobei die Struktur (100) an der Komponente des Absorptionsartikels (20) unterhalb der Struktur (100) befestigt ist, wobei die Struktur (100) eine Längsabmessung und eine Lateralabmessung und eine Dicke aufweist, und umfassend ein oder mehrere elastische Elemente (195, 196); und/oder wobei die Struktur (100) mit einem oder mehreren elastischen Elementen (195, 196) verbunden ist; wobei die Struktur (100) eine erste und eine zweite Schicht (110, 120) umfasst, wobei jede Schicht eine innere (111, 121) und eine äußere Oberfläche (112, 122), eine Längsabmessung, die parallel zu der Längsabmessung der Struktur (100) liegt, die durch erste und zweite beabstandete laterale Ränder begrenzt ist, sowie eine Lateralabmessung aufweist, die parallel zu der Lateralabmessung der Struktur (100) ist, die durch zwei beabstandete Längsränder begrenzt ist, wobei sich die erste und die zweite Schicht (110, 120) mindestens teilweise überlappen, wobei die zweite Schicht (120) in Bezug auf die erste Schicht (110) in der Lage ist, sich entlang der Längsabmessung nach Freisetzung einer Kontraktionskraft, die von dem einen oder den mehreren gedehnten elastischen Elementen entlang der Längsabmessung bereitgestellt wurde, zu verschieben,

wobei die Struktur (100) ferner Bänder (130) umfasst, die zwischen der ersten und der zweiten Schicht (110, 120) in mindestens einem Teil des Bereichs bereitgestellt sind, wo sich die erste und die zweite Schicht überlappen,

wobei jedes Band (130) eine Längsabmessung, die durch zwei beabstandete laterale Bandränder (134) begrenzt ist, sowie eine Lateralabmessung aufweist, die durch zwei beabstandete Längsbandränder begrenzt ist,

wobei jedes Band (130) an der inneren Oberfläche (111) der ersten Schicht (110) mit einem Abschnitt an oder benachbart zu einem der lateralen Ränder (134) des Bands in einem ersten Bandbefestigungsbereich (135) befestigt ist und an der inneren Oberfläche (121) der zweiten Schicht (120) mit einem Abschnitt an oder benachbart zu dem anderen lateralen Rand (134) des Bands in einem zweiten Bandbefestigungsbereich (136) befestigt ist, wobei der Bereich jedes Bands zwischen dem ersten und dem zweiten Bandbefestigungsbereich (135, 136) einen freien Zwischenabschnitt (137) bildet, wobei die Bänder (130) voneinander entlang der Längsabmessung der Struktur (100) beabstandet sind und die Befestigung aller Bänder (130) an der ersten und der zweiten Schicht (110, 120) in dem ersten und dem zweiten Bandbefestigungsbereich (135, 136) derart ist, dass die freien Zwischenabschnitte (137) der Bänder (130) nach Freisetzung einer Kontraktionskraft, die von dem einen oder den mehreren gedehnten elastischen Elementen entlang der Längsabmessung der Struktur (100) bereitgestellt wurde, in der Lage sind, sich von einer zunächst flachen Konfiguration in eine aufrechte Konfiguration umzuwandeln, so dass die Struktur insgesamt von einer zunächst flachen Konfiguration in eine aufrechte Konfiguration umgewandelt wird, wobei die Aufrichtung in der Richtung erfolgt, die senkrecht zu der Längs- und der Lateralabmessung der Struktur ist, so dass eine Dicke gesteigert wird,

wobei sich die Struktur (100) in einer flachen Konfiguration befindet, wenn der Absorptionsartikel (20) in einen flachen Zustand gebracht wird, wobei das eine oder die mehreren elastischen Elemente (195, 196) entlang der Längsabmessung der Struktur (100) gedehnt sind,

wodurch eine Kontraktion des einen oder der mehreren elastischen Elemente (195, 106) eine Kraft entlang der Längsabmessung der Struktur (100) anlegt, so dass die Struktur in die aufrechte, dreidimensionale Konfiguration umgewandelt wird, wobei die Struktur im Vergleich zu der Dicke der Struktur in ihrer flachen Konfiguration in ihrer aufrechten Konfiguration eine gesteigerte Dicke aufweist und die aufrechte Struktur die Oberschicht (24) weg vom Absorptionskern (28) beabstandet.

2. Absorptionsartikel nach Anspruch 1, wobei die flüssigkeitsdurchlässige Oberschicht (24) in der Längs- und/oder Querabmessung des Absorptionsartikels (20) verlängerbar ist, so dass die flüssigkeitsdurchlässige Oberschicht (24) eine Umwandlung der Struktur (100) von ihrer flachen Konfiguration in ihre aufrechte Konfiguration nicht behindert.

3. Absorptionsartikel nach einem der vorstehenden Ansprüche, wobei eine sekundäre Oberschicht unterhalb der Struktur und oberhalb des Absorptionskerns angeordnet ist.

4. Absorptionsartikel (20) mit einer Längsabmessung und einer Querabmessung und umfassend
eine flüssigkeitsdurchlässige Oberschicht (24), die eine körperseitige Oberfläche und eine bekleidungsseitige Oberfläche aufweist,
eine flüssigkeitsundurchlässige Unterschicht (26); und
einen Absorptionskern (28), der zwischen der Oberschicht (24) und der Unterschicht (26) angeordnet ist; wobei der Absorptionsartikel (20) ferner eine Struktur (100) umfasst, die über der Oberschicht (24) angeordnet und an der trägerseitigen Oberfläche der Oberschicht befestigt ist, wobei die Struktur eine Längsabmessung und eine Lateralabmessung und eine Dicke aufweist, und umfassend ein oder mehrere elastische Elemente (195, 196); und/oder wobei die Struktur mit einem oder mehreren elastischen Elementen verbunden ist;
wobei die Struktur (100) eine erste und eine zweite Schicht (110, 120) umfasst, wobei jede Schicht eine innere (111, 121) und eine äußere Oberfläche (112, 122), eine Längsabmessung, die parallel zu der Längsabmessung der Struktur (100) ist, die durch erste und zweite beabstandete laterale Ränder begrenzt ist, sowie eine Lateralabmessung aufweist, die parallel zu der Lateralabmessung der Struktur (100) ist, die durch zwei beabstandete Längsränder begrenzt ist, wobei sich die erste und die zweite Schicht (110, 120) mindestens teilweise überlappen, wobei die zweite Schicht (120) in Bezug auf die erste Schicht (110) in der Lage ist, sich entlang der Längsabmessung nach Freisetzung einer Kontraktionskraft, die von dem einen oder den mehreren gedehnten elastischen Elementen entlang der Längsabmessung bereitgestellt wurde, zu verschieben,
wobei die Struktur (100) ferner Bänder (130) umfasst, die zwischen der ersten und der zweiten Schicht (110, 120) in mindestens einem Teil des Bereichs bereitgestellt sind, wo sich die erste und die zweite Schicht überlappen,
wobei jedes Band (130) eine Längsabmessung, die durch zwei beabstandete laterale Bandränder (134) begrenzt ist, und eine Lateralabmessung aufweist, die durch zwei beabstandete Längsbandränder begrenzt ist,
wobei jedes Band (130) an der inneren Oberfläche (111) der ersten Schicht (110) mit einem Abschnitt an oder benachbart zu den lateralen Rändern (134) des Bands in einem ersten Bandbefestigungsbereich (135) befestigt ist und an der inneren Oberfläche (121) der zweiten Schicht (120) mit einem Abschnitt an oder benachbart zu dem weiteren lateralen Rand (134) des Bands in einem zweiten Bandbefestigungsbereich (136) befestigt ist, wobei der Bereich jedes Bands zwischen dem ersten und dem zweiten Bandbefestigungsbereich (135, 136) einen freien Zwischenabschnitt (137) bildet,
wobei die Bänder (130) voneinander entlang der Längsabmessung der Struktur (100) beabstandet sind und die Befestigung aller Bänder (130) an der ersten und der zweiten Schicht (110, 120) in dem ersten und dem zweiten Bandbefestigungsbereich (135, 136) derart ist, dass sich die freien Zwischenabschnitte (137) der Bänder (130) nach Freisetzung einer Kontraktionskraft, die von dem einen oder den mehreren gedehnten elastischen Elementen entlang der Längsabmessung der Struktur (100) bereitgestellt wurde, in der Lage sind, sich von einer zunächst flachen Konfiguration in eine aufrechte Konfiguration umzuwandeln, so dass die Struktur insgesamt von einer zunächst flachen Konfiguration in eine aufrechte Konfiguration umgewandelt wird, wobei die Aufrichtung in der Richtung erfolgt, die senkrecht zu der Längs- und der Lateralabmessung der Struktur ist, so dass die Dicke erhöht wird,
wobei sich die Struktur (100) in einer flachen Konfiguration befindet, wenn der Absorptionsartikel (20) in einen flachen Zustand gebracht wird, wobei das eine oder die mehreren elastischen Elemente (195, 196) entlang der Längsabmessung der Struktur gedehnt sind,
wodurch eine Kontraktion des einen oder der mehreren elastischen Elemente (195, 106) eine Kraft entlang der Längsabmessung der Struktur (100) anlegt, so dass die Struktur in die aufrechte, dreidimensionale Konfiguration umgewandelt wird, wobei die Struktur im Vergleich zu der Dicke der Struktur in ihrer flachen Konfiguration in ihrer aufrechten Konfiguration eine gesteigerte Dicke aufweist.

5. Absorptionsartikel nach einem der vorstehenden Ansprüche, wobei die äußere Oberfläche (112) der ersten Schicht (110) an einem der Folgenden befestigt ist: der Oberschicht, der sekundären Oberschicht unterhalb der Struktur, dem Aufnahmesystem unterhalb der Struktur und/oder dem Absorptionskern unterhalb der Struktur.

6. Absorptionsartikel nach Anspruch 1 bis 4, wobei die erste Schicht (110) der Struktur durch einen beliebigen der Folgenden gebildet ist: einen Abschnitt der Oberschicht, einen Abschnitt der sekundären Oberschicht unterhalb der Struktur, einen Abschnitt des Aufnahmesystems unterhalb der Struktur oder einen Abschnitt des Absorptionskerns unterhalb der Struktur.

7. Absorptionsartikel nach einem der vorstehenden Ansprüche, wobei das eine oder die mehreren elastischen Elemente (195, 196) einen oder mehrere Abschnitte der zweiten Schicht (120) bilden.

8. Absorptionsartikel nach einem der vorstehenden Ansprüche, wobei das eine oder die mehreren elastischen Elemente (195, 196) an der zweiten Schicht (120) befestigt sind.

9. Absorptionsartikel nach einem der vorstehenden Ansprüche, wobei die zweite Schicht (120) an der ersten Schicht (110), an der Oberschicht, an der sekundären Oberschicht, an dem Aufnahmesystem und/oder an dem Absorptionskern in Längsrichtung außerhalb des zweiten Bandbefestigungsbereichs (136), der sich am nächsten zu dem ersten lateralen Rand der Struktur befindet, dauerhaft befestigt ist (197) und wobei das eine oder die mehreren elastischen Elemente (196) mindestens einen Abschnitt der zweiten Schicht (120) zwischen dem zweiten Bandbefestigungsbereich (136), der sich am nächsten zu dem ersten lateralen Rand der Struktur befindet, und der Befestigung (197) der zweiten Schicht (120) an der ersten Schicht (110), der Oberschicht, an der sekundären Oberschicht, dem Aufnahmesystem und/oder dem Absorptionskern bilden.

10. Absorptionsartikel nach einem der vorstehenden Ansprüche, wobei die zweite Schicht (120) an der ersten Schicht (110), an der Oberschicht, an der sekundären Oberschicht, an dem Aufnahmesystem und/oder an dem Absorptionskern in Längsrichtung außerhalb des zweiten Bandbefestigungsbereichs (136), der sich am nächsten zu dem ersten lateralen Rand der Struktur (100) befindet, dauerhaft befestigt ist (197) und wobei das eine oder die mehreren elastischen Elemente (196) mindestens zwischen dem zweiten Bandbefestigungsbereich (136), der sich am nächsten zu dem ersten lateralen Rand der Struktur befindet, und der Befestigung der zweiten Schicht (120) an der ersten Schicht (110), der Oberschicht, an der sekundären Oberschicht, dem Aufnahmesystem und/oder dem Absorptionskern an der zweiten Schicht (120) befestigt sind.

11. Absorptionsartikel nach einem der vorstehenden Ansprüche, wobei, in der flachen Konfiguration der Struktur (100), die zweite Schicht (120) an der ersten Schicht (110), an der Oberschicht, an der sekundären Oberschicht, an dem Aufnahmesystem und/oder an dem Absorptionskern in einer Position, die sich auf der Seite des einen oder der mehreren elastischen Elemente (195) befindet, entlang der Längsabmessung der Struktur lösbar befestigt (198) ist, die der Seite gegenüberliegt, an der die zweite Schicht (120) an der ersten Schicht, an der Oberschicht, an der sekundären Oberschicht, an dem Aufnahmesystem und/oder an dem Absorptionskern dauerhaft befestigt ist (197); und wobei die Ablösung des lösbaren Befestigung (198) ermöglicht, dass sich das eine oder die mehreren elastischen Elemente (195) kontrahieren und dadurch die Struktur von ihrer flachen Konfiguration in ihre aufrechte Konfiguration umwandeln.

12. Absorptionsartikel nach einem der vorstehenden Ansprüche, wobei das eine oder die mehreren elastischen Elemente (195) (a) einen Abschnitt(e) der zweiten Schicht (120) zwischen benachbarten zweiten Bandbefestigungsbereichen (136) bilden.

13. Absorptionsartikel nach einem der vorstehenden Ansprüche, wobei das eine oder die mehreren elastischen Elemente an der zweiten Schicht (120) zwischen benachbarten zweiten Bandbefestigungsbereichen (136) befestigt sind.

14. Absorptionsartikel nach Anspruch 12 oder 13, wobei die zweite Schicht (120) an der ersten Schicht (110), an der Oberschicht, an der sekundären Oberschicht, an dem Aufnahmesystem und/oder an dem Absorptionskern auf beiden Seiten des einen oder der mehreren elastischen Elemente (195) entlang der Längsabmessung der Struktur lösbar befestigt ist (198) und wobei die Ablösung der lösbaren Befestigungen (198) ermöglicht, dass sich das eine oder die mehreren elastischen Elemente (195) kontrahieren und dadurch die Struktur von ihrer flachen Konfiguration in ihre aufrechte Konfiguration umwandeln.

15. Absorptionsartikel nach einem der vorstehenden Ansprüche, wobei, in der Struktur, die Bänder (130), in ihrer aufrechten Konfiguration, entweder eine Z-ähnliche Form, eine C-ähnliche Form, eine T-ähnliche Form oder eine Doppel-T-ähnliche Form annehmen.

16. Absorptionsartikel nach einem der vorstehenden Ansprüche, wobei die Struktur eine oder mehrere Stopphilfe(n) (180, 190) umfasst, welche die maximale Verschiebung der zweiten Schicht (120) in Bezug auf die erste Schicht (110) entlang der Längsabmessung definiert bzw. definieren, wenn die Kraft entlang der Längsabmessung aufgrund einer Kontraktion des einen oder der mehreren elastischen Elemente (195, 196) angelegt wird, wobei die maximale Verschiebung, die durch die Stopphilfe (180, 190) definiert wird, geringer als die maximale Verschiebung ist, die von den Bändern (130) ohne eine solche Stopphilfe bereitgestellt wird.

**17.** Absorptionsartikel nach Anspruch 16, wobei die eine oder die mehreren Stopphilfen (180, 190) ausgewählt sind aus der Gruppe bestehend aus:

a. einer Schicht-an-Schicht-Stopphilfe (180), die sich von der ersten Schicht zur zweiten Schicht erstreckt und an der ersten Schicht in einem ersten Schicht-an-Schicht-Stopphilfe-Befestigungsbereich befestigt ist und ferner an der zweiten Schicht in einem zweiten Schicht-an-Schicht-Stopphilfe-Befestigungsbereich befestigt ist, wobei die Schicht-an-Schicht-Stopphilfe mit einem Schicht-an-Schicht-Stopphilfe-Spielraum zwischen dem ersten Schicht-an-Schicht-Stopphilfe-Befestigungsbereich und dem zweiten Schicht-an-Schicht-Stopphilfe-Befestigungsbereich versehen ist, wenn sich die Struktur in ihrer zunächst flachen Konfiguration befindet, wobei der Spielraum in der Lage ist, in einen geraden Zustand gebracht und/oder ausgebreitet zu werden, wenn die Struktur in ihre aufrechte Konfiguration überführt wird; und

b. einer Schicht-an-Band-Stopphilfe (190), die sich von der ersten Schicht oder der zweiten Schicht zu einem der Bänder erstreckt und an der ersten oder der zweiten Schicht in einem ersten Schicht-an-Band-Stopphilfe-Befestigungsbereich befestigt ist und an dem Band in einem zweiten Schicht-an-Band-Stopphilfe-Befestigungsbereich befestigt ist, wobei die Schicht-an-Band-Stopphilfe mit einem Schicht-an-Band-Stopphilfe-Spielraum zwischen dem ersten Schicht-an-Band-Stopphilfe-Befestigungsbereich und dem zweiten Schicht-an-Band-Stopphilfe-Befestigungsbereich versehen ist, wenn sich die Struktur in ihrer zunächst flachen Konfiguration befindet, wobei der Spielraum in der Lage ist, in einen geraden Zustand gebracht und/oder ausgebreitet zu werden, wenn die Struktur in ihre aufrechte Konfiguration überführt wird; und

c. einer umhüllenden Stopphilfe, die mindestens einen Abschnitt der ersten Schicht und der zweiten Schicht und der Bänder zwischen der ersten und der zweiten Schicht umschließt, wobei die umhüllende Stopphilfe an der ersten Schicht, der zweiten Schicht und/oder einem oder mehreren Bändern in mindestens einem umhüllenden Stopphilfe-Befestigungsbereich befestigt ist und wobei die umhüllende Stopphilfe ferner an sich selbst befestigt ist, um eine geschlossene Schleife mit einem definierten Umfang um mindestens einen Abschnitt der ersten und der zweiten Schicht und der Bänder zwischen der ersten und der zweiten Schicht zu bilden, wobei der Umfang der umhüllenden Stopphilfe die maximale Dicke der Struktur in ihrer aufrechten Konfiguration definiert; wobei die Dicke senkrecht zu der Lateral- und der Längsabmessung der Struktur ist; und

d. beliebigen Kombinationen von a) bis c).

**18.** Absorptionsartikel nach einem der vorstehenden Ansprüche, wobei in der Struktur die Regionen, in denen sich die Bänder befinden, und die Regionen zwischen benachbarten Bändern einen Modul von etwa 0,01 N/mm$^2$ bis etwa 0,3 N/mm$^2$ aufweisen, wenn sie gemäß dem hierin dargelegten Prüfverfahren gemessen werden.

## Revendications

**1.** Article absorbant (20) ayant une dimension longitudinale et une dimension transversale et comprenant
une feuille de dessus perméable aux liquides (24),
une feuille de fond imperméable aux liquides (26) ; et
une âme absorbante (28) disposée entre la feuille de dessus (24) et la feuille de fond (26) ; l'article absorbant (20) comprenant, en outre, une structure (100) positionnée au-dessus de l'âme absorbante (28) et en dessous de la feuille de dessus (24) dans lequel la structure (100) est attachée au composant de l'article absorbant (20) en dessous de la structure (100), la structure (100) ayant une dimension longitudinale et une dimension latérale et une épaisseur, et comprenant un ou plusieurs éléments élastiques (195, 196) ; et/ou la structure (100) est associée à un ou plusieurs éléments élastiques (195, 196);
dans lequel la structure (100) comprend une première et une deuxième couches (110, 120), chaque couche ayant une surface interne (111, 121) et une surface externe (112, 122), une dimension longitudinale parallèle à la dimension longitudinale de la structure (100) confinée par des premier et deuxième bords latéraux espacés, et une dimension latérale parallèle à la dimension latérale de la structure (100) confinée par deux bords longitudinaux espacés, dans lequel les première et deuxième couches (110, 120) se recouvrent au moins partiellement, dans lequel la deuxième couche (120) est capable de se décaler par rapport à la première couche (110) le long de la dimension longitudinale de la structure lors de la libération d'une force de contraction, qui est conférée par le ou les éléments élastiques étirés le long de la dimension longitudinale,
la structure (100) comprenant, en outre, des ligaments (130) fournis entre les première et deuxième couches (110, 120) dans au moins une partie de la région où les première et deuxième couches se recouvrent,
chaque ligament (130) a une dimension longitudinale confinée par deux bords latéraux espacés (134) du ligament, et une dimension latérale confinée par deux bords longitudinaux espacés du ligament,
chaque ligament (130) étant attaché à la surface interne (111) de la première couche (110) par une partie située au niveau ou à côté de l'un des bords latéraux (134) du ligament dans une première région d'attachement de ligament (135) et étant attaché à la surface interne (121) de la deuxième couche (120) par une partie située au niveau ou à côté de l'autre bord latéral (134) du ligament dans une deuxième région d'attachement de ligament (136), la région de chaque ligament entre les première et deuxième régions d'attachement de ligament (135, 136) formant une partie intermédiaire libre (137), les ligaments (130) étant espacés les uns des autres le long de la dimension longitudinale de la structure (100), et l'attachement de tous les ligaments (130) aux première et deuxième couches (110, 120) dans les première et deuxième régions d'attachement de ligament (135, 136) est tel que les parties intermédiaires libres (137) des ligaments (130) peuvent passer d'une configuration plate initiale à une configuration dressée lors de la libération d'une force de contraction, qui est conférée par le ou les éléments élastiques étirés le long de la dimension longitudinale de la structure (100), faisant ainsi passer la structure dans son ensemble d'une configuration plate initiale à une configuration dressée, dans lequel le redressement s'effectue dans la direction perpendiculaire aux dimensions longitudinale et latérale de la structure, qui augmente ainsi en épaisseur, la structure (100) se trouvant dans la configuration plate lorsque l'article absorbant (20) est aplati, dans lequel les un ou plusieurs éléments élastiques (195, 196) sont étirés le long de la dimension longitudinale de la structure (100), de sorte que la contraction des un ou plusieurs éléments élastiques (195, 106) exerce une force le long de la dimension longitudinale de la structure (100), faisant ainsi passer la structure dans la configuration tridimensionnelle dressée, dans lequel la structure a une plus grande épaisseur dans sa configuration dressée que dans sa configuration plate, la structure dressée éloignant la feuille de dessus (24) de l'âme absorbante (28).

2. Article absorbant selon la revendication 1, dans lequel la feuille de dessus perméable aux liquides (24) est extensible dans les dimensions longitudinale et/ou transversale de l'article absorbant (20), de telle sorte que la feuille de dessus perméable aux liquides (24) ne gêne pas le passage de la structure (100) de sa configuration plate à sa configuration dressée.

3. Article absorbant selon l'une quelconque des revendications précédentes, dans lequel une feuille de dessus secondaire est positionnée en dessous de la structure et au-dessous de l'âme absorbante.

4. Article absorbant (20) ayant une dimension longitudinale et une dimension transversale et comprenant
   une feuille de dessus perméable aux liquides (24), ayant une surface faisant face au corps et une surface tournée vers le vêtement,
   une feuille de fond imperméable aux liquides (26) ; et
   une âme absorbante (28) disposée entre la feuille de dessus (24) et la feuille de fond (26) ; l'article absorbant (20) comprenant, en outre, une structure (100) positionnée au-dessus de la feuille de dessus (24) et attachée à la surface tournée vers le porteur de la feuille de dessus, la structure ayant une dimension longitudinale et une dimension latérale et une épaisseur, et comprenant un ou plusieurs éléments élastiques (195, 196) ; et/ou la structure étant associée à un ou plusieurs éléments élastiques ;
   dans lequel la structure (100) comprend une première et une deuxième couches (110, 120), chaque couche ayant une surface interne (111, 121) et une surface externe (112, 122), une dimension longitudinale parallèle à la dimension longitudinale de la structure (100) confinée par des premier et deuxième bords latéraux espacés, et une dimension latérale parallèle à la dimension latérale de la structure (100) confinée par deux bords longitudinaux espacés, dans lequel les première et deuxième couches (110, 120) se recouvrent au moins partiellement, dans lequel la deuxième couche (120) est capable de se décaler par rapport à la première couche (110) le long de la dimension longitudinale de la structure lors de la libération d'une force de contraction, qui est conférée par le ou les éléments élastiques étirés le long de la dimension longitudinale,
   la structure (100) comprenant, en outre, des ligaments (130) fournis entre les première et deuxième couches (110, 120) dans au moins une partie de la région où les première et deuxième couches se recouvrent,
   chaque ligament (130) a une dimension longitudinale confinée par deux bords latéraux espacés (134) du ligament, et une dimension latérale confinée par deux bords longitudinaux espacés du ligament,
   chaque ligament (130) étant attaché à la surface interne (111) de la première couche (110) par une partie située au niveau ou à côté de l'un des bords latéraux (134) du ligament dans une première région d'attachement de ligament (135) et étant attaché à la surface interne (121) de la deuxième couche (120) par une partie située au niveau ou à côté de l'autre bord latéral (134) du ligament dans une deuxième région d'attachement de ligament (136), la région de chaque ligament entre les première et deuxième régions d'attachement de ligament (135, 136) formant une partie intermédiaire libre (137),
   les ligaments (130) étant espacés les uns des autres le long de la dimension longitudinale de la structure (100), et l'attachement de tous les ligaments (130) aux première et deuxième couches (110, 120) dans les première et deuxième régions d'attachement de ligament (135, 136) est tel que les parties intermédiaires libres (137) des ligaments (130) peuvent passer d'une configuration plate initiale à une configuration dressée lors de la libération d'une force de contraction, qui est conférée par le ou les éléments élastiques étirés le long de la dimension longitudinale de la structure (100), faisant ainsi passer la structure dans son ensemble d'une configuration plate initiale à une configuration dressée, dans lequel le redressement s'effectue dans la direction perpendiculaire aux dimensions longitudinale et latérale de la structure, qui augmente ainsi en épaisseur,

la structure (100) se trouvant dans la configuration plate lorsque l'article absorbant (20) est aplati, dans lequel les un ou plusieurs éléments élastiques (195, 196) sont étirés le long de la dimension longitudinale de la structure, de sorte que la contraction des un ou plusieurs éléments élastiques (195, 106) exerce une force le long de la dimension longitudinale de la structure (100), faisant ainsi passer la structure dans la configuration tridimensionnelle dressée, dans lequel la structure a une plus grande épaisseur dans sa configuration dressée que dans sa configuration plate.

5. Article absorbant selon l'une quelconque des revendications précédentes, dans lequel la surface externe (112) de la première couche (110) est attachée à l'un quelconque des éléments suivants : la feuille de dessus, la feuille de dessus secondaire en dessous de la structure, le système de recueil en dessous de la structure et/ou l'âme absorbante en dessous de la structure.

6. Article absorbant selon l'une quelconque des revendications 1 à 4, dans lequel la première couche (110) de la structure est formée de l'un quelconque des éléments suivants : une partie de la feuille de dessus, une partie de la feuille de dessus secondaire en dessous de la structure, une partie du système de recueil en dessous de la structure ou une partie de l'âme absorbante en dessous de la structure.

7. Article absorbant selon l'une quelconque des revendications précédentes, dans lequel les un ou plusieurs éléments élastiques (195, 196) forment une ou plusieurs parties de la deuxième couche (120).

8. Article absorbant selon l'une quelconque des revendications précédentes, dans lequel les un ou plusieurs éléments élastiques (195, 196) sont attachés à la deuxième couche (120).

9. Article absorbant selon l'une quelconque des revendications précédentes, dans lequel la deuxième couche (120) est attachée de façon permanente (197) à la première couche (110), à la feuille de dessus, à la feuille de dessus secondaire, au système de recueil et/ou à l'âme absorbante, longitudinalement à l'extérieur par rapport à la deuxième région d'attachement de ligament (136) qui est la plus proche du premier bord latéral de la structure, et dans lequel les un ou plusieurs éléments élastiques (196) forment au moins une partie de la deuxième couche (120) entre la deuxième région d'attachement de ligament (136) qui est la plus proche du premier bord latéral de la structure et l'attachement (197) de la deuxième couche (120) à la première couche (110), à la feuille de dessus, à la feuille de dessus secondaire, au système de recueil et/ou à l'âme absorbante.

10. Article absorbant selon l'une quelconque des revendications précédentes, dans lequel la deuxième couche (120) est attachée de façon permanente (197) à la première couche (110), à la feuille de dessus, à la feuille de dessus secondaire, au système de recueil et/ou à l'âme absorbante, longitudinalement à l'extérieur par rapport à la deuxième région d'attachement de ligament (136) qui est la plus proche du premier bord latéral de la structure (100), et dans lequel les un ou plusieurs éléments élastiques (196) sont attachés à la deuxième couche (120) au moins entre la deuxième région d'attachement de ligament (136) qui est la plus proche du premier bord latéral de la structure et l'attachement de la deuxième couche (120) à la première couche (110), à la feuille de dessus, à la feuille de dessus secondaire, au système de recueil et/ou à l'âme absorbante.

11. Article absorbant selon l'une quelconque des revendications précédentes, dans lequel, dans la configuration plate de la structure (100), la deuxième couche (120) est attachée de façon libérable (198) à la première couche (110), à la feuille de dessus, à la feuille de dessus secondaire, au système de recueil et/ou à l'âme absorbante, à un endroit qui se trouve du côté des un ou plusieurs éléments élastiques (195) le long de la dimension longitudinale de la structure qui est opposée au côté où la deuxième couche (120) est attachée de façon permanente (197) à la première couche, à la feuille de dessus, à la feuille de dessus secondaire, au système de recueil et/ou à l'âme absorbante, et dans lequel la libération de l'attachement libérable (198) permet aux un ou plusieurs éléments élastiques (195) de se contracter et de faire ainsi passer la structure de sa configuration plate à sa configuration dressée.

12. Article absorbant selon l'une quelconque des revendications précédentes, dans lequel les un ou plusieurs éléments élastiques (195) forment une ou plusieurs parties de la deuxième couche (120) entre des deuxièmes régions d'attachement de ligament (136) adjacentes.

**13.** Article absorbant selon l'une quelconque des revendications précédentes, dans lequel les un ou plusieurs éléments élastiques sont attachés à la deuxième couche (120) entre des deuxièmes régions d'attachement de ligament (136) adjacentes.

**14.** Article absorbant selon la revendication 12 ou 13, dans lequel la deuxième couche (120) est attachée de façon libérable (198) à la première couche (110), à la feuille de dessus, à la feuille de dessus secondaire, au système de recueil et/ou à l'âme absorbante, des deux côtés des un ou plusieurs éléments élastiques (195) le long de la dimension longitudinale de la structure, et dans lequel la libération des attachements libérables (198) permet aux un ou plusieurs éléments élastiques (195) de se contracter et de faire ainsi passer la structure de sa configuration plate à sa configuration dressée.

**15.** Article absorbant selon l'une quelconque des revendications précédentes, dans lequel, dans la structure, les ligaments (130), dans leur configuration dressée, adoptent une forme en Z, une forme en C, une forme en T ou une forme en double T.

**16.** Article absorbant selon l'une quelconque des revendications précédentes, dans lequel la structure comprend un ou plusieurs auxiliaires d'arrêt (180, 190) qui définissent le décalage maximal de la deuxième couche (120) par rapport à la première couche (110) le long de la dimension longitudinale lorsque la force le long de la dimension longitudinale s'exerce du fait de la contraction du ou des éléments élastiques (195, 196), dans lequel le décalage maximal défini par l'auxiliaire d'arrêt (180, 190) est inférieur au décalage maximal permis par les ligaments (130) en l'absence d'un tel auxiliaire d'arrêt.

**17.** Article absorbant selon la revendication 16, dans lequel les un ou plusieurs auxiliaires d'arrêt (180, 190) sont choisis dans le groupe constitué de :

a. un auxiliaire d'arrêt de couche à couche (180) s'étendant de la première couche à la deuxième couche externe et étant attaché à la première couche dans une première région d'attachement d'auxiliaire d'arrêt de couche à couche et étant, en outre, attaché à la deuxième couche dans une deuxième région d'attachement d'auxiliaire d'arrêt de couche à couche, dans lequel l'auxiliaire d'arrêt de couche à couche est muni d'une marge d'auxiliaire d'arrêt de couche à couche entre la première région d'attachement d'auxiliaire d'arrêt de couche à couche et la deuxième région d'attachement d'auxiliaire d'arrêt de couche à couche lorsque la structure se trouve dans sa configuration plate initiale, ladite marge pouvant se redresser et/ou s'étendre lorsque la structure passe dans sa configuration dressée ; et

b. un auxiliaire d'arrêt de couche à ligament (190) s'étendant de la première ou deuxième couche à l'un des ligaments et étant attaché à la première ou deuxième couche dans une première région d'attachement d'auxiliaire d'arrêt de couche à ligament et étant attaché au ligament dans une deuxième région d'attachement d'auxiliaire d'arrêt de couche à ligament, dans lequel l'auxiliaire d'arrêt de couche à ligament est muni d'une marge d'auxiliaire d'arrêt de couche à ligament entre la première région d'attachement d'auxiliaire d'arrêt de couche à ligament et la deuxième région d'attachement d'auxiliaire d'arrêt de couche à ligament lorsque la structure se trouve dans sa configuration plate initiale, ladite marge pouvant se redresser et/ou s'étendre lorsque la structure passe dans sa configuration dressée ; et

c. un auxiliaire d'arrêt enveloppant entourant au moins une partie des première et deuxième couches et des ligaments entre les première et deuxième couches, dans lequel l'auxiliaire d'arrêt enveloppant est attaché à la première couche, à la deuxième couche et/ou à un ou plusieurs ligaments dans au moins une région d'attachement d'auxiliaire d'arrêt enveloppant, et dans lequel l'auxiliaire d'arrêt enveloppant est, en outre, attaché à lui-même pour former une boucle fermée de circonférence définie autour d'au moins une partie des première et deuxième couches et des ligaments entre les première et deuxième couches, dans lequel la circonférence de l'auxiliaire d'arrêt enveloppant définit l'épaisseur maximale de la structure dans sa configuration dressée ; dans lequel l'épaisseur est perpendiculaire aux dimensions latérale et longitudinale de la structure ; et

d. toute combinaison de a) à c).

**18.** Article absorbant selon l'une quelconque des revendications précédentes, dans lequel, dans la structure, les zones où se trouvent les ligaments et les zones entre ligaments voisins ont un module d'environ 0,01 N/mm$^2$ à environ 0,3 N/mm$^2$, mesuré selon la méthode d'essai décrite aux présentes.

Fig. 1A

Fig. 1B

Fig. 2

Fig. 3

Fig. 4A

Fig. 4B

Fig. 5A

Fig. 5B

EP 3 067 029 B1

**Fig. 6**

136  132  130  120  134  122
131  150  135  134  110  140  112  111

**Fig. 7**

136  120  134  132  122  121
130  131  134  135  110  137  112  111

Fig. 8a

Fig. 8b

Fig. 8c

Fig. 9a

Fig. 9b

Fig. 9c

EP 3 067 029 B1

Fig. 10a

Fig. 10b

Fig. 10c

EP 3 067 029 B1

**Fig. 11a**

**Fig. 11b**

**Fig. 12a**

**Fig. 12b**

**Fig. 13**

Fig. 14a

Fig. 14b

Fig. 14c

Fig. 15a

Fig. 15b

Fig. 15c

Fig. 16a

Fig. 16b

Fig. 16c

EP 3 067 029 B1

Fig. 17a

Fig. 17b

Fig. 18

**Fig. 19**

196  120  136  34

197  135  130  110  198

**Fig. 20**

136  120  100

196

197  130  135  110

**Fig. 21**

136  120  100

196

197  135  110  130

EP 3 067 029 B1

**Fig. 22**

**Fig. 23**

**Fig. 24**

# Fig. 25

Fig. 26

**Fig. 27**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2005267434 A
- WO 9517150 A2
- US 2005074584 A
- EP 0985395 A1
- US 2006142723 A
- EP 1132066 A
- US 3860003 A
- US 5221274 A
- US 5554145 A
- US 5569234 A
- US 5580411 A
- US 6004306 A